# EUROPEAN PATENT SPECIFICATION

(11) **EP 2 578 193 B1**
(45) Date of publication and mention of the grant of the patent: **23.03.2016**
(21) Application number: 11789771.0
(22) Date of filing: 31.05.2011
(51) Int. Cl.: A61F 13/00, A61F 13/02

(54) **SURFACE SHEET FOR WOUND DRESSING AND WOUND DRESSING**
OBERFLÄCHENFOLIE FÜR EINEN WUNDVERBAND UND WUNDVERBAND
FEUILLE DE SURFACE POUR UN PANSEMENT POUR PLAIES ET PANSEMENT POUR PLAIES

(30) Priority: 01.06.2010 JP 2010126338
(43) Date of publication of application: 10.04.2013
(73) Proprietor: Zuiko Corporation, Settsu-Shi Osaka 566-0045 (JP)
(72) Inventor: KURATA, Shuhei, Settsu-shi Osaka 566-0045 (JP)
(74) Representative: Teipel, Stephan
(86) International application number: PCT/JP2011/062427
(87) International publication number: WO 2011/152368

(56) References cited:
- EP-A1- 1 640 023
- EP-A1- 2 039 328
- WO-A1-01/60296
- WO-A1-2005/000372
- WO-A1-2008/004380
- JP-A- 2003 522 596
- JP-A- 2006 517 427
- JP-A- 2010 131 163

## Description

### TECHNICAL FIELD

The present invention relates to a wound dressing suitable for treating wounds, such as a burn, a bed sore, a contusion, a cut, an abrasion and an ulcer.

### BACKGROUND ART

In recent years it has turned out to be effective in healing a wound not to dry the wound surface but to keep it in a moist environment. In particular, it has been discovered that components contained in exudate from a wound site promote the healing of the wound and it therefore is effective to employ a treatment method in which a wound is not disinfected but treated in a moist environment created by the exudate (hereinafter sometimes referred to as "moist healing"). Based on the discovery, various types of wound dressings applicable to such a therapeutic method have been developed.

In order to effectively perform moist healing, it is important to retain a moderate amount of exudate from a wound site and thereby maintain a moderate moist environment on the wound surface. A wound dressing is therefore required to have a function of retaining a moderate amount of exudate on the wound surface instead of rapidly absorbing it. In moist healing, however, a wound dressing is firmly fixed to the skin in order to maintain a moist environment and a closed area is formed on the wound surface. Due to this closed area, when additional exudate oozes and is superfluously retained, the excess exudate compresses the wound surface, causing "undermining" (a phenomenon in which the skin on the wound site is hollowed by the pressure from the exudate). For this reason, a wound dressing is required to have a function of removing from the wound surface a moderate amount of the exudate.

In addition, if the material in contact with the wound site lacks breathability and sticks tight to the wound surface, removing the wound dressing may damage the healed or healing wound again. For this reason, it is required that a wound dressing does not stick tight to a wound surface so as to be easily peeled off after use, but that the wound dressing is firmly fixed to the wound site to maintain a moist environment for the treatment of the wound during use.

As a conventional wound dressing, for example, JP 7-80020 A discloses a dressing utilizing a porous film in which a hydrophilic substance is dispersed or which is coated with a hydrophilic substance. However, in this wound dressing, only the exudate-removing function is improved by utilizing a hydrophilic porous film, and the wound dressing is not suitable for the objective of retaining a moderate amount of exudate on the wound surface.

JP 10-151184 A discloses a wound dressing applied onto an ulcer surface, the dressing being made of a cotton, a knitted or woven fabric, a nonwoven fabric, or the like which contains a chitin-chitosan cellulose mixed fiber and to which a hydrocolloid agent is applied if desired. However, in this wound dressing, emphasis is placed on the function of absorbing exudate, and the function of retaining a moderate amount of the exudate on the wound surface is only insufficiently achieved. Further, it has been pointed out that a prolonged direct contact of the hydrocolloid agent of the wound dressing with the skin may cause a skin redness or a heat rash.

EP 2 039 328 A1 discloses a wound dressing suitable for a method to treat a wound while maintaining a moist environment created by exudate from the wound site.

EP 1 640 023 A1 discloses a wound dressing that is applied on a wound site so as to cover the wound site, comprising a permeable layer, an adsorptive layer and an impermeable layer, the layers being integrally stacked together so that the adsorptive layer is sandwiched between the permeable layer and the impermeable layer.

In order to solve the above problems of conventional wound dressings, the inventor has developed a wound dressing having a sheet material which exerts an initial water pressure resistance as a permeable layer in contact with a wound site, and already applied for an international patent for the dressing (see WO 2005/000372 and WO 2008/004380). The wound dressing has outstanding functions for use in a method for treating a wound while maintaining a moist environment created by the exudate from the wound site.

However, there has been a demand for the development of a further improved wound dressing, i.e., a wound dressing having the following features:
(i) having a sufficient function of retaining a moderate amount of the exudate on the wound surface, while preventing the exudate from leaking,
(ii) preventing unnecessary spread of the exudate, which spread causes irritation of unwounded normal skin,
(iii) not sticking tight to the wound surface so as to be easily peeled off after use, but firmly fixed to the wound site to maintain a moist environment for the treatment of the wound during use,
(iv) causing no skin redness or heat rash,
(v) causing no offensive smell, and
(vi) comprising a thin flexible material so as to fit various shapes of wound surfaces and not to compress the wound surface.

### CITATION LIST

### PATENT LITERATURE

Patent Literature 1: JP 7-80020 A (Claim 1, Paragraphs 0010 and 0011)
Patent Literature 2: JP 10-151184 A (Claims)
Patent Literature 3: WO 2005/000372 (Abstract)
Patent Literature 4: WO 2008/004380

### SUMMARY OF INVENTION

### TECHNICAL PROBLEM

An object of the present invention is to provide a further improved wound dressing suitable for a method for treating a wound while maintaining a moist environment created by exudate from the wound site.

### SOLUTION TO PROBLEM

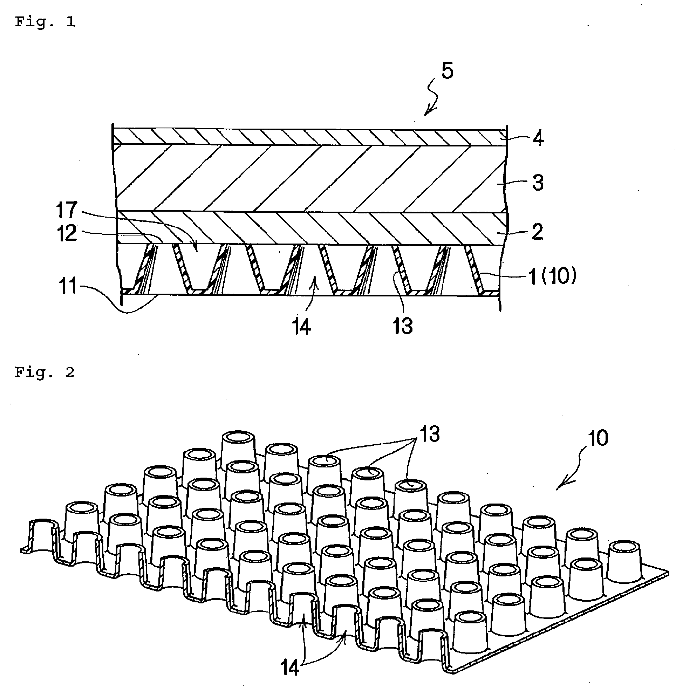

The present invention for solving the above problems will be described with reference to, for example, Figs. 1 to 17 illustrating embodiments of the present invention. The present invention encompasses the following aspects.
[1] A surface sheet for a wound dressing, the surface sheet being disposed on at least a portion of the wound dressing (5) which portion is to face a wound site and comprising a resin sheet material having a first surface (11) which is to face the wound site (15), a second surface (12) which is the opposite surface to the first surface, and a large number of penetration pores (13) penetrating through the sheet material from the first surface (11) to the second surface (12) in the thickness direction,
   the penetration pores (13) allowing the permeation of a liquid from the first surface (11) to the second surface (12), and the first surface (11) being hydrophobic, and wherein the opening area of the penetration pores (13) being between 280 and 1400 µm in diameter, the density of the penetration pores (13) being between 50 and 400 pores/cm², the pore opening diameter ratio of the first surface (11) to the second surface (12) being between 1.1 and 1.8, the depth of the penetration pores (13) being between 100 and 2000 µm, the capacity of the retaining space formed within the penetration pores (13) being between 0.015 and 0.55 micro-liters, and the sheet material comprises a low-density polyethylene resin material.
[2] The surface sheet for a wound dressing according to the above [1], wherein the first surface (11) has a contact angle with physiological saline of 85 degrees or more.
[3] The surface sheet for a wound dressing according to the above [1] or [2], wherein the first surface (11) has a surface tension of 40 dyne/cm or less.
[4] The surface sheet for a wound dressing according to any of the above [1] to [3], wherein the first surface (11) is coated with one or more water-repellent materials selected from the group consisting of silicone, polyurethane, a styrene-butadiene-styrene block copolymer, a tetrafluoroethylene-hexafluoropropylene copolymer, a tetrafluoroethylene-perfluoroalkylvinyl ether copolymer, and polytetrafluoroethylene.
[5] A wound dressing comprising at least two layers, a liquid-permeable layer (1) and an absorbing retaining layer (3),
   the two layers being stacked in the following order from the side to be used in contact with a wound site (15): the liquid-permeable layer (1) and the absorbing retaining layer (3),
   the liquid-permeable layer (1) comprising the surface sheet (10) according to any of the above [1] to [4], and
   the absorbing retaining layer (3) comprising a sheet material capable of absorbing and retaining water.
[6] The wound dressing according to the above [5], wherein a second liquid-permeable layer (1a) is further stacked on the opposite surface of the absorbing retaining layer (3) to the surface on which the liquid-permeable layer (1) is disposed.
[7] The wound dressing according to the above [5], wherein a protective layer (4) is further provided on the opposite surface of the absorbing retaining layer (3) to the surface which is closer to the wound site, and the protective layer (4) comprises a resin film, a woven fabric, a knitted fabric, or a nonwoven fabric.
[8] The wound dressing according to the above [7], wherein the protective layer (4), which has a wider area than those of the other layers, covers all of the other layers and extends outward beyond the edges of the other layers to form a peripheral portion (6), and the peripheral portion (6) has an adhesive area (7) on at least a part of the surface on which the other layers are stacked.
[9] The wound dressing according to the above [8], wherein the peripheral portion (6) of the protective layer (4) has a non-adhesive area (8), which is other than the adhesive area (7).
[10] The wound dressing according to the above [8], wherein a part of the peripheral portion (6) of the protective layer (4) outside the edges of the other layers is omitted.
[11] The wound dressing according to the above [8], wherein the peripheral portion (6) of the protective layer (4) has a slit (9) or a small pore along the perimeters of the other layers.
[12] The wound dressing according to any of the above [5] to [11], wherein a liquid-permeation restricting layer (2) is disposed between the liquid-permeable layer (1, 1a) and the absorbing retaining layer (3),
   the liquid-permeation restricting layer (2) comprises a microporous film, a woven fabric, a knitted fabric, or a nonwoven fabric, each of which comprises a hydrophobic material, and
   through the liquid-permeation restricting layer (2), a liquid is allowed to migrate from the liquid-permeable layer (1) to the absorbing retaining layer (3).
[13] The wound dressing according to the above [12], wherein the liquid-permeation restricting layer (2) comprises a polyolefin resin and has a permeability measured according to JIS L 1096 of 5 to 2000 cm³/cm²·S and a water repellency measured according to JIS L 1092 of grade 3 or higher.
[14] The wound dressing according to the above [12] or [13], wherein the liquid-permeation restricting layer (2) comprises a nonwoven fabric comprising a polypropylene fiber.
[15] The wound dressing according to any of the above [5] to [14], wherein the absorbing retaining layer (3) comprises an airlaid nonwoven fabric.
[16] The wound dressing according to any of the above [5] to [15], wherein the absorbing retaining layer (3) comprises fluff pulp.
[17] The wound dressing according to the above [16] , wherein the absorbing retaining layer (3) further comprises a super absorbent polymer, and the weight ratio of the super absorbent polymer and the fluff pulp is 10:90 to 25:75.
[18] The wound dressing according to the above [17], wherein the super absorbent polymer is a sodium polyacrylate polymer.
[19] The wound dressing according to any of the above [5] to [18], wherein the absorbing retaining layer (3) is partially not joined to an adjacent layer which is on the side closer to the wound site.
[20] The wound dressing according to the above [19], wherein the absorbing retaining layer (3) is not integrated with the liquid-permeable layer (1) and is movable along the second surface (12) of the liquid-permeable layer (1).
[21] A wound dressing comprising a liquid-permeable layer (1) comprising the surface sheet (10) according to any of the above [1] to [4].
[22] The wound dressing according to the above [21], wherein a liquid-permeation restricting layer (2) is stacked on the second surface (12) of the surface sheet (10),
   the liquid-permeation restricting layer (2) comprises a microporous film, a woven fabric, a knitted fabric, or a nonwoven fabric, each of which comprises a hydrophobic material, and
   the liquid-permeation restricting layer (2) allows the permeation of a liquid in the thickness direction.
[23] The wound dressing according to the above [22], wherein the liquid-permeation restricting layer (2) comprises a polyolefin resin and has a permeability measured according to JIS L 1096 of 5 to 2000 cm³/cm²·S and a water repellency measured according to JIS L 1092 of grade 3 or higher.
[24] The wound dressing according to the above [22] or [23], wherein the liquid-permeation restricting layer (2) comprises a nonwoven fabric comprising a polypropylene fiber.
[25] The wound dressing according to any of the above [5] to [24], comprising an adhesive layer (21) on the opposite surface to the surface which is to face the wound site (15).

### ADVANTAGEOUS EFFECTS OF INVENTION

The present invention having the above structure exerts the following effects.
(1) The surface sheet of the present invention is provided with a large number of penetration pores on the first surface. Hence, a wound dressing utilizing the surface sheet, when applied to a wound site to cover it, can excellently retain exudate oozing from the wound. In addition, since the first surface is hydrophobic, the wound dressing does not suck up oozing exudate rapidly but retains the exudate to prevent it from spreading over a larger area and from leaking while maintaining a moist environment. In this way, by retaining the exudate in the area around the wound site, the therapeutic effect can be enhanced while unnecessary spread of the exudate, which causes irritation of unwounded normal skin, can be prevented. Therefore a wound dressing utilizing the surface sheet of the present invention is suitable for the treatment of various types of wounds and most suitable for the prevention and treatment of a bed sore in particular.
(2) The surface sheet comprises a resin sheet material having a large number of penetration pores. Hence, the surface sheet does not stick tight to a wound site. Further, a wound dressing utilizing the surface sheet can firmly fix its circumferential part around a wound site to maintain a moist environment for the treatment of the wound. In addition, the wound dressing can be easily peeled off after use, which greatly alleviates the pain at the time of exchange.
(3) The surface sheet comprising a resin sheet material is disposed on the surface which is to face a wound site. Hence, unlike the above conventional art in which a hydrophilic colloid or the like is disposed on the surface which is to face a wound site, a wound dressing utilizing the surface sheet of the present invention will cause neither skin redness nor heat rash even after prolonged attachment.
(4) A wound dressing utilizing the surface sheet of the present invention comprises a resin sheet material on the surface which is to face a wound site. That is, the wound dressing can be made of a thin flexible material. The wound dressing is therefore capable of fitting various shapes of wound surfaces and causes no compression of the wound surfaces.
(5) While the surface sheet maintains a moist environment by retaining exudate between the surface sheet and a wound surface or within the penetration pores, the penetration pores allow the permeation of a liquid from the first surface to the second surface. Hence, there is no risk that excessive exudate is retained on the wound site. In addition, since the surface sheet comprises a resin sheet material, the exudate which has permeated through the penetration pores hardly flows back to the wound site. Therefore, even if various germs grow in the exudate which has passed through the surface sheet and been retained for a long time, there is no risk that the exudate flows back to the wound site and thus decay of the wound site and occurrence of ammonia smell are efficiently prevented.

### BRIEF DESCRIPTION OF DRAWINGS

Fig. 1 is an enlarged schematic sectional view illustrating the principal part of the wound dressing of a first embodiment of the present invention.
Fig. 2 is a fragmented schematic perspective view illustrating a surface sheet of the first embodiment.
Fig. 3 is a corresponding view of Fig. 1, illustrating the state in which exudate is caught in the liquid-permeable layer in the first embodiment.
Fig. 4 is a corresponding view of Fig. 1, illustrating the state in which exudate is entering the liquid-permeation restricting layer in the first embodiment.
Fig. 5 is a corresponding view of Fig. 1, illustrating the state in which exudate is received by the second surface of the liquid-permeable layer in the first embodiment.
Fig. 6 is a partially fragmented schematic plan view illustrating the wound dressing of the first embodiment seen from the surface-sheet side.
Fig. 7 is a corresponding view of Fig. 6, illustrating Modified Example 1 of the present invention.
Fig. 8 is a corresponding view of Fig. 6, illustrating Modified Example 2 of the present invention.
Fig. 9 is a corresponding view of Fig. 6, illustrating Modified Example 3 of the present invention.
Fig. 10 is a corresponding view of Fig. 6, illustrating Modified Example 4 of the present invention.
Fig. 11 is a corresponding view of Fig. 6, illustrating Modified Example 5 of the present invention.
Fig. 12 is a schematic perspective view illustrating a process for producing the wound dressing of the present invention.
Fig. 13 is a corresponding view of Fig. 1, illustrating a second embodiment of the present invention.
Fig. 14 is a corresponding view of Fig. 1, illustrating a third embodiment of the present invention.
Fig. 15 is a corresponding view of Fig. 1, illustrating a fourth embodiment of the present invention.
Fig. 16 is a corresponding view of Fig. 1, illustrating the application of the fourth embodiment to a diaper.
Fig. 17 is a partially fragmented perspective view of the wound dressing, illustrating a fifth embodiment.

### DESCRIPTION OF EMBODIMENTS

The wound dressing of the present invention is a dressing to be attached to a wound site to cover it, and can be applied to a method for treating a wound while maintaining a moist environment created by exudate from the wound. In the present invention, the term "wound" broadly means skin injuries including a burn, a bed sore, a contusion, a cut, an abrasion, an ulcer, a postoperative wound, and the like. In the present invention, the term "sheet material" broadly means both porous and nonporous sheet materials including, for example, a resin film, a fabric, a nonwoven fabric, a net, and the like, and the thickness thereof is not particularly limited.

Hereinafter, the configuration of the wound dressing of the present invention will be described referring to the drawings as needed.

Fig. 1 is a schematic sectional view showing a first embodiment of the wound dressing of the present invention.

The wound dressing (5) of the first embodiment comprises at least the following two layers in the following order from the side to be used in contact with a wound site, a liquid-permeable layer (1) formed of a surface sheet (10), and an absorbing retaining layer (3) formed of a sheet material which can absorb water and retain it. As shown in Fig. 1, the wound dressing (5) further may comprises, if desired, a liquid-permeation restricting layer (2) disposed between the liquid-permeable layer (1) and the absorbing retaining layer (3), and a protective layer (4) disposed on the opposite surface of the absorbing retaining layer (3) to the surface on which the liquid-permeation restricting layer (2) is disposed. These layers are integrally stacked. During use, the liquid-permeable layer (1) is in contact with a wound site.

The surface sheet (10) forming the liquid-permeable layer (1) is disposed on the portion which is to face a wound site. The surface sheet (10) comprises, for example, as shown in Figs. 1 and 2, a resin sheet material having a first surface (11) which is to face a wound site, a second surface (12) which is the opposite surface to the first surface, and a large number of penetration pores (13) penetrating through the sheet material from the first surface (11) to the second surface (12) in the thickness direction. The penetration pores (13) allow the permeation of a liquid from the first surface (11) to the second surface (12). The first surface (11) is hydrophobic.

The entire surface of the absorbing retaining layer (3) may be wholly joined to an adjacent layer which is on the side closer to a wound site, namely, in the first embodiment, the liquid-permeation restricting layer (2). Alternatively, the absorbing retaining layer (3) may be partially joined to the liquid-permeation restricting layer (2). For example, only the circumferential part of the absorbing retaining layer (3) may be joined to the liquid-permeable layer (1) or the liquid-permeation restricting layer (2), and in this case the center part and the like thereof are free from adhesion to these layers.

In the wound dressing of the present invention, the phrase "the layers are integrally stacked" means that the layers are stacked on top of each other by at least partially joining each layer and that this stacked condition is maintained without separation of the layers in normal use unless an external force is applied to forcibly separate the layers. Examples of the joining means for stacking include, for example, in addition to adhesion using adhesives such as a hot-melt adhesive, fusion by heat sealing, and embossing, but are not limited thereto.

Hereinafter, each layer will be specifically described in detail.

### Liquid-permeable layer

In order to heal a wound, it is sufficient that exudate is retained in the area around the wound site. It is not preferred that the exudate spreads beyond the area around the wound site. This is because, in such an area with the spread exudate, unwounded normal skin may be irritated and the wound site may be further expanded, which may retard the healing.

The liquid-permeable layer (1) is provided for the main purpose of retaining exudate at the place where it oozes from a wound to prevent the exudate from spreading over a larger area while maintaining a moist environment by not sucking up the exudate rapidly. In this way, the liquid-permeable layer (1) accelerates the healing of a wound.

The liquid-permeable layer (1) is formed of the surface sheet (10) made of a resin sheet material and has a large number of the penetration pores (13) penetrating through the layer from the first surface (11) to the second surface (12) in the thickness direction. Each of the penetration pores (13) is preferably independent from each other and the liquid-permeable layer (1) preferably does not have any path inside through which water can flow in the in-plane direction. A large number of the penetration pores (13) open on the first surface (11) of the liquid-permeable layer (1), thereby preventing the liquid-permeable layer (1) from sticking tight to the wound site.

As shown in Figs. 1 and 2, the sheet material forming the liquid-permeable layer (1) is formed to have concavities and convexities. The first surface (11) refers to one surface of the liquid-permeable layer (1) which surface is to be in contact with a plane on the side on which a wound site is present. The second surface (12) refers to the other surface of the liquid-permeable layer (1) which surface is to be in contact with a plane on the side on which a wound site is not present.

The penetration pores (13) may be in any shape such as a cylindrical shape, a barrel shape, and a tsuzumi (Japanese hand drum) shape and preferably, for example, in the shape of a "tapered pore" whose diameter gradually decreases from the first surface (11) side to the second surface (12) side, as shown in Figs. 1 and 2.

The opening area of the penetration pores (13) on the first surface (11) which is to face a wound site corresponds to a circle 280 to 1400 µm in diameter.

Since the penetration pores (13) are tapered, the opening area of the pores on the second surface (12) is smaller than that on the first surface (11). In comparison in terms of the diameter of the circle corresponding to the opening area (hereinafter referred to as "pore opening diameter"), the pore opening diameter on the first surface (11) is 1.1 to 1.8 times larger than that on the second surface (12), preferably 1.2 to 1.5 times larger.

The density of the penetration pores (13) is 50 to 400 pores/cm², preferably 60 to 325 pores/cm². The opening ratio of the penetration pores (13) on the first surface (11) is preferably 15 to 60%.

The depth of the penetration pores (13), i.e. , the distance between the first surface (11) and the second surface (12) or the thickness of the liquid-permeable layer (1) is 100 to 2000 µm, preferably 250 to 500 µm.

The density, pore opening ratio and depth of the penetration pores (13) within the above ranges enables the formation of a moderate capacity of retaining space (14) between the wound site and the second surface (12) for the retention of a moderate amount of the exudate on the wound surface, and prevents the exudate from spreading in the in-plane direction of the wound site.

The capacity of the retaining space (14) formed within the penetration pores (13) is 0.015 to 0.55 µL per penetration pore, preferably 0.030 to 0.45 µL per penetration pore, most preferably 0.040 to 0.35 µL per penetration pore. At least the first surface (11) of the liquid-permeable layer (1) is hydrophobic and therefore the liquid-permeable layer (1) can be prevented from sticking excessively tight to a wound site and can be easily peeled off the wound site after use. While the penetration pores (13) allow the permeation of a liquid from the first surface (11) to the second surface (12), the liquid-permeable layer (1) in which at least the first surface (11) is hydrophobic can restrict the migration of the exudate, through the penetration pores (13), to the absorbing retaining layer (3) having a moisture absorbing property (liquid absorbing property), thereby excellently retaining the exudate between the liquid-permeable layer (1) and the wound site, which accelerates the healing of the wound.

The liquid-permeable layer (1) is not limited to a particular material etc. as long as the first surface (11) which is to face at least a wound site is hydrophobic.

However, in view of retaining exudate in an amount necessary for treatment between the wound site and the liquid-permeable layer (1) and of facilitating easier peeling of the wound dressing (5) after use, at least the first surface (11) has preferably a dynamic contact angle (hereinafter sometimes simply referred to as "contact angle") with physiological saline of 85 degrees or more. Further, in view of facilitating further easier peeling of the wound dressing (5) after use, the contact angle with physiological saline is more preferably 95 degrees or more, especially preferably 100 degrees or more. The "contact angle" used in the present invention refers to the value measured by the θ/2 method.

The "contact angle" is measured according to, for example, JIS K 2396. In particular, the measurement is performed, for example, as follows. A 1.5- to 2-cm square is cut out from a sheet material sample and placed on the measuring stage of a contact angle measurement instrument (model: FTA-100, First Ten Angstrom, Inc.). From a syringe installed in the instrument, 1.5 µL of a calibration standard droplet sample is dispensed and made into contact with the sample piece. One, three, five, and 10 minutes later, the dynamic contact angle is measured by the sessile drop method (droplet supply rate: 0.5 µL/second; droplet volume: 1.5 µL) and analyzed with the contact angle measurement instrument.

In view of holding the wound dressing (5) to a wound site so that exudate in an amount necessary for the treatment of the wound is retained and of facilitating easier peeling of the wound dressing (5) after use, the liquid-permeable layer (1) is preferably made of a material having a dynamic surface tension (hereinafter sometimes simply referred to as "surface tension") of 40 dyne/cm or less, more preferably 35 dyne/cm or less. For further easier peeling after use, particularly preferred is 32 dyne/cm or less. The surface tension more than 40 dyne/cm is not preferable because such surface tension does not reduce adhesion between the liquid-permeable layer (1) and the wound site, which results in difficulties in peeling off the wound dressing (5) after use and in smoothly exchanging it. The surface tension may be adjusted to 40 dyne/cm or less by surface treatment, such as the addition of a known additive, corona treatment or plasma treatment, and the like.

In particular, the "surface tension" is measured according to, for example, the following procedure. A 1.5- to 2-cm square is cut out from a sheet material sample and placed on the measuring stage of a contact angle measurement instrument (model: FTA-100, First Ten Angstrom, Inc.). From a syringe installed in the above instrument, 1.5 µL of a test mixed solution is dispensed, and the surface tension is determined by the pendant drop method and analyzed with the contact angle measurement instrument.

The surface sheet (10) forming the liquid-permeable layer (1) is preferably a resin film, in particular, a porous film made by punching a large number of pores in a resin film. The resin material used to form the surface sheet (10) is not limited to a particular material unless the effects of the present invention are hindered and the sheet material comprises a low-density polyethylene resin. For the purpose of retaining a moderate amount, for example, 5 µL/cm² or more etc. of moisture in the space between the wound site and the wound dressing (5) in order to prevent the wound site from drying, the resin material is preferably a resin having a contact angle with physiological saline of 85 degrees or more. Such a resin is exemplified by a polyolefin resin, a silicone resin, a polytetrafluoroethylene resin, and a polyurethane resin. Among these, especially preferred is a polypropylene resin having a contact angle with physiological saline of 91 degrees. That is, the surface sheet (10) forming the liquid-permeable layer (1) is preferably a porous polyolefin resin film having a contact angle with physiological saline of 85 degrees or more, more preferably a porous polypropylene resin film. Preferably, the penetration pores (13) which are present on the film are tapered pores as described above.

Since surfactant treatment on the surface of the liquid-permeable layer (1) will reduce the above contact angle, the surface is preferably not treated with a surfactant. In addition, neither polyethylene terephthalate resin nor a polyvinylidene chloride resin is preferred as a material for the surface sheet (10) forming the liquid-permeable layer (1) because the surface tension of these resins is more than 40 dyne/cm.

The liquid-permeable layer (1) has hydrophobicity on at least the first surface (11) as described above. This hydrophobicity is not necessarily based only on the properties of the resin material and may be given by water-repellent treatment as needed.

For example, by an appropriate water-repellent treatment, a desired hydrophobicity can be given to even a polyolefin resin (for example, a polyethylene resin) having a contact angle with physiological saline of less than 85 degrees, a polyamide resin such as nylon 6 and nylon 66, a polystyrene resin (contact angle with physiological saline: 84 degrees), and the like.

In this case, preferably employed is a high-density polyethylene resin (density range: 930 to 970 kg/m³) or a low-density polyethylene resin (density range: 910 to 930 kg/m³), and among these especially preferred is a low-density polyethylene resin. This is because these resins are flexible and therefore suitable for forming a closed area to fit the shape and irregularity of a wound site, and allow easy formation of penetration pores and easy performance of a water-repellent treatment for a desired contact angle.

The water-repellent treatment is not limited to a particular treatment method. Examples of the treatment method include a method in which a repellent containing a known water-repellent material is coated by a known coating method (for example, spin coating, dip coating, vacuum deposition, CVD coating, etc.), treatment of the first surface etc. with fluorine plasma, a method for forming fine concavities and convexities on the first surface etc., and the like.

The water-repellent material is not particularly limited and examples thereof include silicone, polyurethane, a styrene-butadiene-styrene block copolymer, a tetrafluoroethylene-hexafluoropropylene copolymer, a tetrafluoroethylene-perfluoroalkylvinyl ether copolymer, polytetrafluoroethylene, and the like. These water-repellent materials may be used alone or in combination of two or more.

The liquid-permeable layer (1) not only allows the permeation of exudate to the second surface (12) through the penetration pores (13) but also restricts the permeation of the exudate due to its hydrophobicity. Preferably, the liquid-permeable layer (1) has a property of becoming liquid-permeable when pressure is applied. In the present invention, the phrase "becoming liquid-permeable when pressure is applied" means that the liquid-permeable layer (1) does not allow the permeation of a liquid until the liquid pressure applied on the layer reaches a predetermined level and, once the liquid pressure becomes higher than the predetermined level, the layer allows the permeation of the liquid.

The property of "becoming liquid-permeable when pressure is applied" can easily be realized when the first surface (11) of the liquid-permeable layer (1) is hydrophobic and when the penetration pores (13) are tapered. The property can also easily be realized when the density of the penetration pores (13) is 50 to 400 pores/cm² and when the pore opening ratio is 15 to 60%.

Preferably, when pressure is applied, the liquid-permeable layer (1) allows the permeation of a liquid through the penetration pores (13). Therefore it is preferred that the liquid-permeable layer (1) substantially does not allow the permeation of a liquid through any part other than the penetration pores (13).

The liquid impermeability of the liquid-permeable layer (1) before pressure is applied can be examined by, for example, the following process. The surface sheet (10) forming the liquid-permeable layer (1) is held horizontally in midair at a fixed height with the use of a metal frame etc., and water is slowly dropped onto the same part of the surface sheet (10) with the use of a pipette from above the sheet. At first, the water does not fall through the surface sheet (10). As the amount of the dropped water increases, the water starts to fall through the surface sheet (10) due to the pressure caused by the weight of the water itself. In this way, by the fact that the water does not fall through the sheet until the amount of the dropped water reaches a predetermined level, it is confirmed that the sheet has a desired liquid impermeability.

The liquid-permeable layer (1) is hydrophobic and therefore works to prevent exudate oozing from a wound from spreading in the surface direction on the wound site.

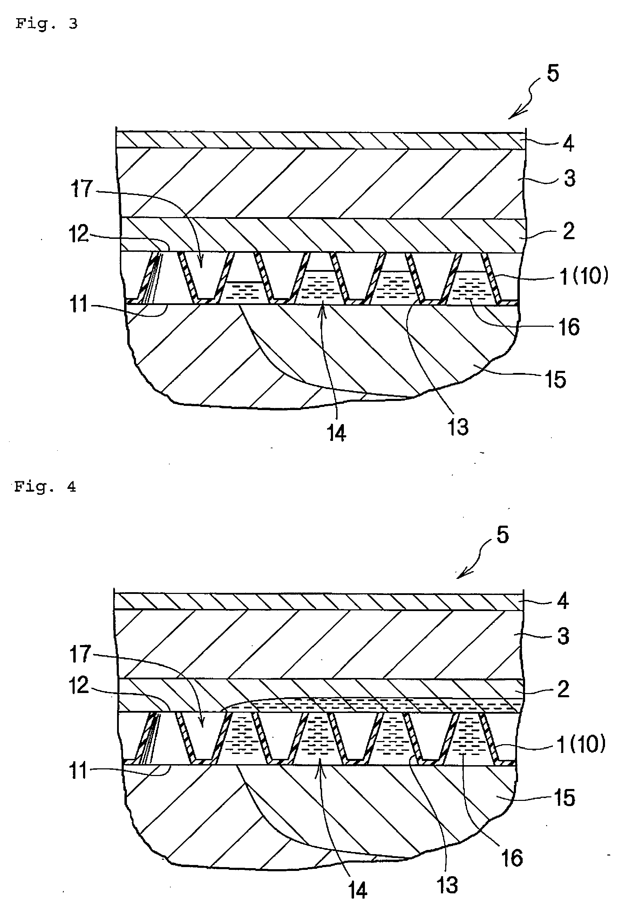

In an early stage, i.e., at a time point when only a short time has passed since the application of the wound dressing (5) of the present invention to a wound site (15) and exudate (16) has started to ooze out from the wound site (15) (the stage before the exudate creates pressure), the exudate (16) permeates halfway through the penetration pores (13), as shown in Fig. 3. Since the first surface of the liquid-permeable layer (1) is hydrophobic, the exudate (16) is caught in the penetration pores (13) and retained between the wound site (15) and the liquid-permeable layer (1) without spreading beyond the area of the wound site in the surface direction on the wound site (15). Thus, a moist environment is maintained on the wound site (15). For the purpose of maintaining a moist environment without the great spread of the exudate (16), preferably the liquid-permeable layer (1) is hydrophobic, the penetration pores (13) are tapered, and the capacity of the retaining space within the penetration pores (13) is 0.015 to 0.55 µL per penetration pore.

In the next stage, when the exudate (16) further oozes out from the wound site (15) and increases its pressure, the exudate (16) permeates through the penetration pores (13) of the liquid-permeable layer (1) and reaches the surface of the liquid-permeation restricting layer (2), and further permeates through the liquid-permeation restricting layer (2) toward the absorbing retaining layer (3), as shown in Fig. 4. At that time, it is preferred that the exudate (16) does not flow back through the penetration pores (13). In particular the exudate (16) which has permeated through the liquid-permeation restricting layer (2) has a risk of the growth of various germs because a substantial time has passed since the oozing of the exudate. For example, if the exudate (16) flows back to the wound site when the wound dressing (5) is compressed by an external force, there also is a risk that these various germs may grow on the wound site (15). For preventing such flow back of the exudate, the penetration pores (13) is preferably tapered.

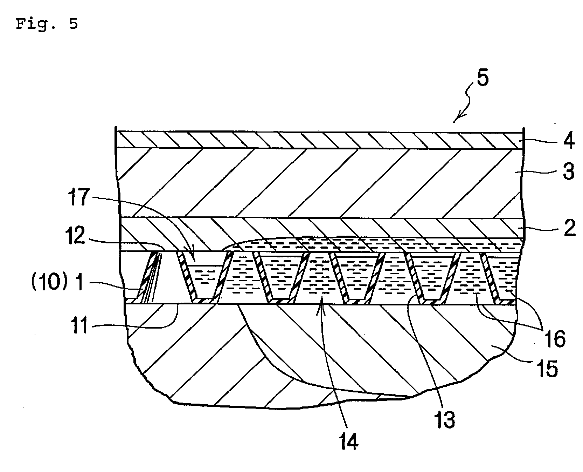

When the liquid-permeable layer (1) has the second surface (12) which is formed to have concavities and convexities as shown in, for example, Fig. 5, the exudate (16) which has reached the liquid-permeation restricting layer (2) is caught in concavities (17) of the second surface (12). This structure is preferable in that it further efficiently prevents the exudate (16) from spreading in the surface direction on the wound site (15).

### Liquid-permeation restricting layer

The wound dressing (5) comprises a liquid-permeation restricting layer (2) between the liquid-permeable layer (1) and the absorbing retaining layer (3), as shown in Fig. 1. The liquid-permeation restricting layer (2) not only has the above property of withstanding the pressure created by the exudate and preventing the permeation of the exudate in an early stage, i.e., at a time point when the exudate has started to ooze out from the wound site after the application of the wound dressing (5) to the wound site, but also has a function of allowing the permeation of the exudate when the amount of the exudate increases and the liquid pressure becomes higher than a predetermined level.

From the above, preferably the liquid-permeation restricting layer (2) has moderate permeability and water repellency. The permeability is determined, for example, with a Frazier type air permeability tester for textiles according to Method A (Frazier type method) described in JIS L 1096. Specifically, the air permeability is determined by attaching a sample onto the Frazier type tester, adjusting the induced draft fan with a rheostat so that the differential barometer reads 125 Pa, and then calculating the amount of air (cm³/cm²·S) passing through the sample from the pressure shown by the vertical barometer at that time and the type of the used orifice, with the use of the table attached to the tester. The air permeability is obtained by calculating the arithmetic mean of 5 measurements. Preferably, the permeability of the liquid-permeation restricting layer (2) is 5 to 2000 cm³/cm²·S.

The water repellency is determined, for example, with a water repellency test apparatus with a spray nozzle having predetermined capability (the ability to spray 250 mL of water for 25 to 30 seconds) according to the water repellency test (spray test) described in JIS L 1092. Specifically, the water repellency is determined by (1) attaching a sample (about 20 cm x 20 cm) onto the sample holding frame of the water repellency test apparatus, and spraying 250 mL of water from the spray nozzle onto the sample over 25 to 30 seconds, (2) removing the holding frame from the stand of the water repellency test apparatus, and performing predetermined operation to let excessive waterdrops fall off the sample, and (3) comparing the wet condition of the sample attached to the holding frame with those of comparison samples in predetermined wet conditions to grade the sample. The predetermined operation in the above (2) refers to an operation of, while holding one side of the above holding frame in a horizontal position, turning the sample face down, gently hitting the opposite side against something hard, and then horizontally rotating it 180 degrees to do the same. In the water repellency test, the temperature is set at 20 ± 2°C, and the water for this test is distilled or deionized water. Preferably, the water repellency of the liquid-permeation restricting layer (2) is grade 3 or higher.

The sheet material forming the liquid-permeation restricting layer (2) may be, for example, a nonwoven fabric, a porous film, a knitted or woven fabric, or the like, which is made of a hydrophobic material such as a polyolefin resin (for example, polypropylene, polyethylene, etc.), a polyester resin (for example, polyethylene terephthalate, polybutylene terephthalate, polypropylene terephthalate, etc.), a polyamide resin (for example, nylon 6, nylon 66, etc.), a polyurethane resin, and the like. Among these preferred is a nonwoven fabric, more preferably a nonwoven fabric made of a polyolefin fiber, most preferably a nonwoven fabric made of a polypropylene fiber.

The nonwoven fabric which can be used for the liquid-permeation restricting layer (2) is not limited to a particular type, and various types of wetlaid or drylaid nonwoven fabrics can be used. Examples thereof include thermally bonded nonwoven fabrics, needle punched nonwoven fabrics, spunlace nonwoven fabrics, spunbonded nonwoven fabrics, meltblown nonwoven fabrics, flashspun nonwoven fabrics, or complex nonwoven fabrics thereof. Examples of the complex nonwoven fabrics include what is called SMS nonwoven fabrics, SMMS nonwoven fabrics, and the like, which are produced by combining a meltblown nonwoven fabric and a spunbonded nonwoven fabric. The above nonwoven fabrics are not limited to fabrics made of a hydrophobic synthetic fiber and may be a water-repellent treated nonwoven fabric made of a hydrophilic fiber such as cotton and rayon.

However, the nonwoven fabric used for the liquid-permeation restricting layer (2) is especially preferably a nonwoven fabric obtained by meltblowing or flashspinning. A meltblown nonwoven fabric and a flashspun nonwoven fabric are made of, for example, a superfine fiber with a fiber diameter of about 20 µm or less. Therefore interfiber voids thereof can be smaller compared to those of a nonwoven fabric obtained by the other manufacturing methods such as spunbonding, even when the weight per areas (basis weight) of these fabrics are the same. Consequently, the meltblown nonwoven fabric and the flashspun nonwoven fabric can efficiently retain exudate from a wound site in the closed space and can easily exert the property of "becoming liquid-permeable when pressure is applied".

Simply stated, the meltblown nonwoven fabric is a fabric which is made of a continuous superfine fiber, and is obtained through elongation and fibrillation of the fiber by blowing high-temperature and high-pressure air to the outlet of a spinning nozzle. The meltblown nonwoven fabric may be formed into an SMS nonwoven fabric, an SMMS nonwoven fabric, or the like by combining with a spunbonded nonwoven fabric in layers and used for the liquid-permeation restricting layer (2).

The flashspun nonwoven fabric is a reticulated nonwoven fabric which is made of a continuous superfine fiber, and is obtained by uniformly dissolving a fiber-forming polymer in a low-boiling solvent under high temperature and high pressure and, while discharging the above solution from a nozzle, rapidly gasifying and expanding only the solvent in order to elongate and solidify the fiber-forming polymer.

The nonwoven fabric used for the liquid-permeation restricting layer (2) may be calendered. The calendering as used herein means pressure processing of a nonwoven fabric with calendering rollers or embossing rollers adjusted to a temperature below the melting point. The calendering thermally fuses part of the fibers forming the nonwoven fabric and crushes interfiber voids, which facilitates retaining exudate from a wound site in the closed space.

### Absorbing retaining layer

The absorbing retaining layer (3) is a layer for absorbing the exudate (16) which has oozed out from a wound site and then has successively permeated through the liquid-permeable layer (1) and the liquid-permeation restricting layer (2). For this function, the absorbing retaining layer (3) is formed of a sheet material capable of absorbing and retaining water. The phrase "capable of absorbing and retaining water" means that when placed in contact with a liquid such as water, the sheet naturally absorbs the water and retains at least some of the water in its voids against gravitational force. That is, when the sheet material retaining the absorbed water is lifted, if some of the absorbed water is still held without falling, it can be said that the sheet material is capable of absorbing and retaining water. Preferably, the absorbing retaining layer (3) is made of a sheet material capable of absorbing water via capillary action, but may be made of a material containing a material capable of binding water and retaining it, such as a super absorbent polymer or the like.

The sheet material capable of absorbing and retaining water used for the absorbing retaining layer (3) may be a sponge-like sheet material, but preferably is a sheet material made of a hydrophilic fiber such as cotton, or a hydrophilically treated fiber. The sheet material is preferably a hydrophilically treated nonwoven fabric, fluff pulp, an airlaid nonwoven fabric, or the like, and these materials may be used in combination.

When the absorbing retaining layer (3) is made of fibers, the constituent fibers are preferably bound together by a binder (adhesive), compression, fusion, or the like to the extent that neither waste fibers nor other minor materials drop off from the cut end when the wound dressing (5) is cut. Therefore fibers with thermal adhesiveness are preferably used as at least part of the constituent fibers.

Especially preferred as a material for the absorbing retaining layer (3) is an airlaid nonwoven fabric. An airlaid nonwoven fabric is a nonwoven fabric obtained by cutting and uniformly dispersing raw material pulp fibers or short fibers into the air, and depositing them on a revolving porous cylinder or a movable screen belt while spraying a water-soluble adhesive thereon for interfiber bonding. A pulp-fiber-based airlaid nonwoven fabric is preferable in that it can easily absorb exudate. Especially preferred is an airlaid nonwoven fabric having a pulp fiber content of about 60 to 95% by weight. As a manufacturing method of these nonwoven fabrics, the DAN-WEB method, the Honshu paper method, or the like may be used.

Preferably, the airlaid nonwoven fabric, in order to maintain a desired strength even after the fabric absorbs exudate, contains synthetic fibers with little strength deterioration in wet conditions, in particular, any of synthetic fibers such as a polyamide fiber including nylon 6, nylon 66, and the like; a polyester fiber including polyethylene terephthalate and the like; a polyolefin fiber including polyethylene, polypropylene, and the like.

The airlaid nonwoven fabric may contain a binder fiber. A binder fiber is a fiber exerting an adhesive property by entirely or partially changing its state through fusion and solidification depending on temperature conditions. Specific examples of the binder fiber include completely fusible fibers containing the following resin alone, a low-melting-point resin such as a polyester resin and a polyamide resin, a polyolefin resin, or the like; core-in-sheath composite fibers containing two resins having different melting points such as a polyethylene resin/polypropylene resin and a low-melting-point polyester resin/polypropylene resin; and side-by-side composite fibers.

The absorbing retaining layer (3) may contain an absorption material such as a high absorbent resin powder, comminution pulp (fluff pulp), or the like, to increase the ability to absorb and retain exudate. The absorption material may be in the form of a fiber, as well as a powder and a granule. In addition, by treating the absorption material with a calcium salt, a hemostatic effect on a wound site can be provided.

The absorption material contained in the absorbing retaining layer (3) provided in the wound dressing (5) may drop off from the cut end when the wound dressing (5) is cut for use. The above absorbing retaining layer (3) is preferably made of an airlaid nonwoven fabric because the components (such as fibers) of the airlaid nonwoven fabric are bonded together with a binder under pressure and consequently, even when the wound dressing (5) is cut for use, the high absorbent resin powder, the comminution pulp (fluff pulp), etc. does not easily drop off.

The absorption material refers to a material which, when placed in contact with a liquid, absorbs the liquid, swells and gels in a short period of time. The absorption material may be a known absorption material and is preferably what is called a super absorbent polymer (hereinafter sometimes referred to as SAP) which is polyacrylic, polysulfonic, starch-based, carboxymethylcellulose-based, polyvinyl alcoholic, maleic anhydride-based, polyacrylamide-based, or polyethylene oxide-based; a high absorbent natural polysaccharide such as alginic acid, dextran, etc.; or the like. In cases where the SAP and fluff pulp are mixed, the weight ratio is preferably SAP:fluff pulp = about 10:90 to 25:75. The SAP in this ratio can reduce the offensive smell from exudate from a wound site and, even when the amount of the exudate is large, can prevent the leakage of the exudate from the wound dressing.

Preferred examples of the polyacrylic SAP include sodium polyacrylate-based SAPs obtainable by copolymerizing acrylic acid, sodium acrylate, and a cross-linking monomer. Examples of the cross-linking monomer include a monomer having two or more unsaturated bonds in a molecule, such as allyl methacrylate, triallyl cyanurate, triallyl isocyanate, ethylene glycol dimethacrylate, propylene glycol diallyl ether, divinylbenzene, diethylene glycol dimethacrylate, 1,6-hexanediol dimethacrylate, and the like. The cross-linking monomer is usually used in an amount of 0.1 to 10% by weight, preferably 0.3 to 7% by weight. The polymerization method is not particularly limited and a known method (for example, aqueous polymerization such as adiabatic polymerization and belt polymerization; or inverse-suspension polymerization such as batch polymerization) can be employed. In aqueous polymerization, a suitable solvent is water, a polymerization catalyst is a known catalyst such as a redox catalyst, a persulfate catalyst, an azo catalyst, and the like, and the monomer concentration at the time of polymerization is preferably 20 to 50% by mass. At the initial polymerization stage, the pH of the liquid is preferably 6 or lower, especially preferably 4 or lower, to increase the molecular weight and the moisture absorbing property of the sodium polyacrylate-based polymer. The sodium polyacrylate-based polymer preferably has a limiting viscosity of 0.10 or higher measured under the following conditions: monomer concentration: 0.5 mol/L; catalyst concentration: 2.85 x 10⁻³ mol/L; and polymerization temperature: 50 ± 0.1°C.

The sheet material forming the absorbing retaining layer (3) may comprises a super absorbent fiber which itself has moisture absorbability equivalent to that of the above super absorbent polymer (SAP), and the absorbing retaining layer (3) may be made of virtually only the super absorbent fiber.

The absorbing retaining layer (3) made of virtually only the super absorbent fiber, even when the wound dressing is cut for use, has less risk of dropping off of the high water absorbent resin, compared with a wound dressing containing a high absorbent resin powder. In cases where the absorbing retaining layer (3) contains the resin powder, as the resin powder absorbs exudate and swells, the absorbing retaining layer (3) may become uneven and the layers of the wound dressing may be easily separated. However, in cases where the absorbing retaining layer (3) is made of virtually only the super absorbent fiber, even when the super absorbent fiber absorbs exudate, the absorbing retaining layer (3) is less likely to become uneven and consequently peeling back of the layers can be prevented. Specific examples of the super water-absorbent fiber include, for example, "Lanseall (trademark) F" by Toyobo Co., Ltd.

When the airlaid nonwoven fabric as the absorbing retaining layer (3) absorbs exudate, the hydrogen bonds between the pulp fibers are broken and water-soluble binders are dissolved, which results in the reduction of interfiber bonding in wet conditions. Especially when the airlaid nonwoven fabric contains the above super absorbent resin and the resin absorbs exudate and converts into a gel, the strength of the airlaid nonwoven fabric reduces due to the gelation, and the gelation increases the resin in volume, which results in physical break of the entanglement and binding between the fibers. Thus the wet strength of the airlaid nonwoven fabric may significantly decline.

For the purpose of preventing such decline in the strength, the airlaid nonwoven fabric, especially when containing the super absorbent resin, preferably contains a water-insoluble filament or a binder fiber, as described above. In this way, interfiber bonding necessary for the absorbing retaining layer (3) can be maintained to prevent the decline in the strength in wet conditions. In addition, even when the absorbing retaining layer absorbs exudate, decline in the strength and peeling back of the layers from the absorbing retaining layer of the wound dressing can be prevented. Therefore the wound dressing can be easily and completely peeled off the wound surface.

Preferably, the absorbing retaining layer (3) is stretchy enough to at least deform along a wound site (15), so that the wound dressing is capable of fitting various shapes of wound surfaces. In order to have such stretchiness, the airlaid nonwoven fabric as the absorbing retaining layer (3) may be provided with flexibility and stretchiness by incisions made in an irregular way through, for example, perforation etc. The term "perforation" as used herein refers to a process of making many pores in a nonwoven fabric. The fibers in an airlaid nonwoven fabric are bound together with an adhesive and consequently the stiffness of the fabric tends to be higher and its flexibility may be impaired. Perforation is preferable in that it makes the airlaid nonwoven fabric softer, which facilitates the fitting of the wound dressing to the surface of the wound site. Perforation also allows the nonwoven fabric to absorb the exudate through the inside of the pores as well. Thus once the nonwoven fabric starts absorbing the exudate, the rate of absorption will become increasingly higher. The cross-section shape of the pores generated by perforation is not particularly limited. The pores may completely penetrate or not penetrate the nonwoven fabric.

The absorbing retaining layer (3) may be a complex sheet. The complex sheet can be obtained by, for example, impregnation of a nonwoven fabric with an acrylic acid monomer followed by polymerization and crosslinking reactions. Another example of the complex sheet can be obtained by, for example, dispersing, in a mixed solvent of an organic catalyst and water, a fibrous material having a higher hydration property (for example, microfibril etc.) and a solid substance having water swellability (for example, various kinds of polysaccharides, a flocculant, the above super absorbent polymer, etc.), flow casting the resulting dispersion liquid onto a support sheet such as a nonwoven fabric, and drying off the dispersion liquid.

The thickness of the absorbing retaining layer (3) is not particularly limited but, in view of the capacity to absorb exudate, the thickness is preferably about 0.4 to 0.8 mm. The weight per area of the absorbing retaining layer (3) is not limited to a particular value but is preferably, for example, about 60 to 170 g/m².

The entire surface of the absorbing retaining layer (3) may be wholly joined to an adjacent layer which is on the side closer to the wound site, namely, in the first embodiment, the liquid-permeation restricting layer (2). In an alternative example, however, only the circumferential part of the absorbing retaining layer (3) is joined to the liquid-permeation restricting layer (2) with an adhesive or by heat sealing, and in this case the center part and the like are free from adhesion to the layer. This structure is preferable in that the migration of exudate to the absorbing retaining layer (3) can be restricted and that the exudate can be excellently retained between the wound site and the liquid-permeable layer (1). In addition, the structure allows the absorbing retaining layer (3) and the adjacent layer which is on the side closer to the wound site (15) easily to slide out of alignment. Consequently, such a wound dressing (5) is totally flexible and excellent in texture. Further, the shear stress between the wound dressing (5) and the wound site (15) is absorbed to alleviate the stress applied to the wound, which is also effective in, for example, preventing a bed sore.

### Protective layer

The protective layer is provided in order to prevent the exudate absorbed by the absorbing retaining layer (3) from spreading outside. The wound dressing (5) of the present invention does not always need to have the protective layer. However, when the wound dressing lacks the protective layer, an alternative sheet material for protection is preferably used in combination to prevent the exudate absorbed by the absorbing retaining layer (3) from spreading outside and soiling, for example, clothes, bedding, or the like.

The protective layer (4) is made of a flexible material to fit along the wound site, and is preferably a resin film, a fabric, a nonwoven fabric, or a combination thereof. Among these, a resin film is especially preferred as the protective layer because of its flexibility and stretchiness, and a capability of preventing the permeation of a liquid.

Preferred as the resin film is a resin film which prevents the permeation of a liquid. Examples thereof include a resin film made of an olefin resin (polyethylene, polypropylene, etc.), a polyester resin, a nylon resin, or the like. A stretch resin film made of a polyurethane resin or the like is also preferably used. The use of a stretch resin film improves the fit of the wound dressing to the skin. The thickness of the resin film is not particularly limited and may be suitably determined in view of the strength, flexibility, and the like.

The use of a resin film which prevents the permeation of a liquid as the protective layer (4) is effective in preventing not only the exudate from spreading outside the wound dressing (5) but also water and dirt from entering from the outside. The use also enables more effective maintenance of a moist environment by preventing the evaporation of the exudate.

As described below, a slit or the like may be provided if needed at a part of the resin film which prevents the permeation of a liquid. In this case, the slit can be a passage for a liquid. Even in this case, however, any part except for the slit prevents the permeation of a liquid. Therefore, even in such an embodiment, the fact remains that the resin film is "a resin film which prevents the permeation of a liquid" unless otherwise noted.

The outer surface of the protective layer (4) may be colored or patterned if desired. For example, coloring the surface in a skin-like color makes it less conspicuous. Conversely, an outstanding pattern, illustration, photograph printing, or the like may be intentionally provided to the surface for fashionability and a playful spirit. The protective layer (4) may be transparent, and this case is advantageous because the condition of the exudate in the internal layers is visible and thus appropriate timing for exchanging the wound dressing is easy to know.

The wound dressing of the present invention is in the form of a laminated sheet and it may be supplied, for example, as a roll of a long sheet which may be cut into a desired size before use. In this case, the protective layer (4) has the same area as those of the other layers (1, 2, 3) and the wound dressing is accordingly attached to a wound site with an adhesive plaster or the like.

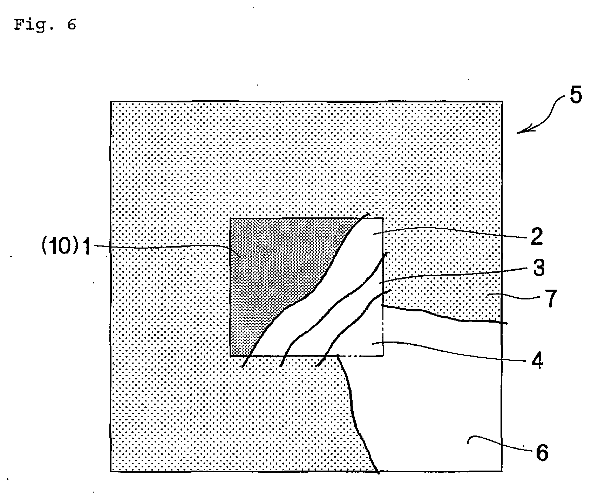

The wound dressing of the present invention may be cut into a convenient size before supplied. In this case, preferably the protective layer (4), which has a wider area than those of the other layers (1, 2, 3), covers all of the other layers (1, 2, 3) and extends outward beyond the edges of the other layers (1, 2, 3) to form a peripheral portion (6), and the peripheral portion (6) has an adhesive area (7) on at least a part of the surface on the side which is to face a wound site. In more detail, for example, as shown in Fig. 6, the liquid-permeable layer (1), the liquid-permeation restricting layer (2), and the absorbing retaining layer (3) made in the same shape are stacked, the protective layer (4) extends outward beyond the edge of the stacked layer to form the peripheral portion (6), and the adhesive area (7) is formed on the surface of the peripheral portion (6).

The adhesive area (7) is provided in order to fix the wound dressing (5) to the skin around the wound site (15). Therefore, the adhesive area (7) is preferably capable of fixing the wound dressing (5) to the skin of the patient and of being easily removed from the skin. The adhesive applied to the adhesive area (7) is preferably a low irritating adhesive not causing skin irritation when in contact with the skin. In particular, preferred adhesive is an acrylic or silicone adhesive. The adhesive used for known adhesive plasters (example: "Band-Aid (trademark)") or the like can be adopted for the adhesive area (7).

### Modified Example

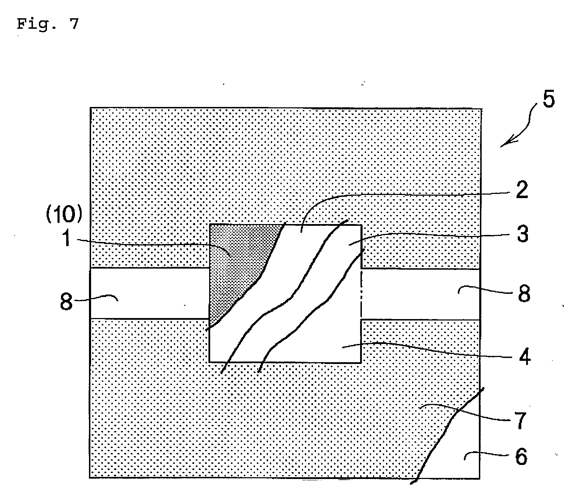

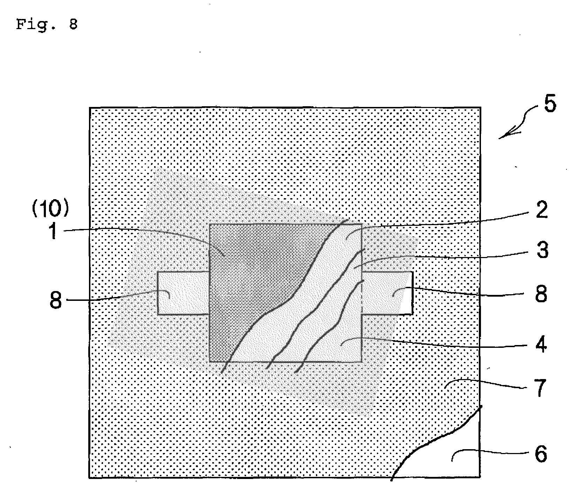

In the above first embodiment, the adhesive area (7) is formed on the entire area of the peripheral portion (6). In the present invention, however, the adhesive area (7) may be provided on part of the peripheral portion (6). That is, part of the adhesive area (7) abutting the perimeters of the other layers (1, 2, 3) may be omitted, as shown in, for example, Modified Example 1 in Fig. 7 or Modified Example 2 in Fig. 8.

That is, in Modified Example 1, a non-adhesive area (8), which is other than the adhesive area (7), extends across the peripheral portion (6) from one edge of the protective layer (4) to the opposite edge of the protective layer (4) passing through the edges of the other layers (1, 2, 3). This Modified Example 1 is preferable in that the non-adhesive area (8), which has no adhesion to the skin, forms an open space which allows air to be supplied to the area around the wound site, thereby preventing the growth of anaerobic bacteria and infections. The protective layer (4) may be formed using a breathable sheet material such as a nonwoven fabric. Alternatively, the protective layer (4) may be formed using a poorly breathable sheet material such as a nonporous resin film. This case is more preferable in that the migration and evaporation of exudate can be prevented as described above.

In Modified Example 2 shown in Fig. 8, the non-adhesive area (8) does not extend to the perimeter of the protective layer (4). In this case, the protective layer (4) is made of a breathable sheet material such as a nonwoven fabric, which allows air to be supplied to the area around the wound site through the protective layer (4) and then the non-adhesive area (8), thereby preventing the growth of anaerobic bacteria and infections.

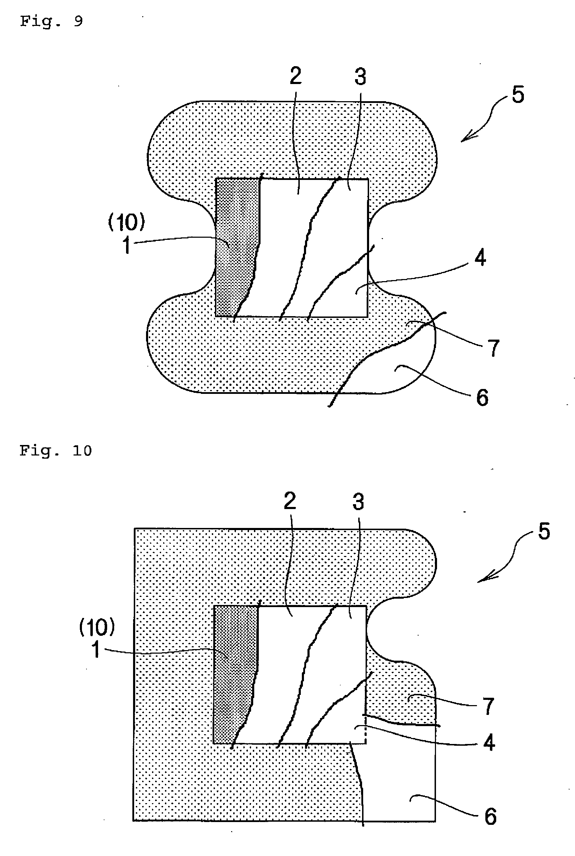

In the above first embodiment, the above peripheral portion (6) is formed all around the perimeters of the other layers (1, 2, 3). In the present invention, however, the peripheral portion (6) of the protective layer (4) may be partially omitted at part of the perimeters of the other layers, as shown in, for example, Modified Example 3 in Fig. 9 or Modified Example 4 in Fig. 10. In this case, the part in which the peripheral portion (6) is omitted forms an open space through which air is supplied to the area around the wound site, thereby preventing the growth of anaerobic bacteria and infections. In such a case where the peripheral portion (6) itself is partially omitted, the adhesive area (7) may be formed throughout the peripheral portion (6).

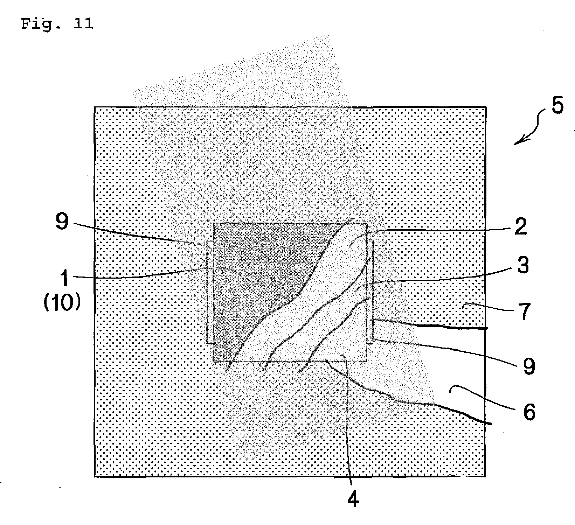

In Modified Example 5 shown in Fig. 11, a slit (9) is formed in the protective layer (4), along the perimeters of the other layers (1, 2, 3). This slit (9), in the same way as in Modified Examples 1 to 4, forms an open space through which air is supplied to the area around the wound site, thereby preventing the growth of anaerobic bacteria and infections.

In Modified Example 5, the slit (9) may be in any form as long as it has an air passage connecting the inside and outside of the protective layer (4), and the slit (9) may be a small pore or the like. The size and number of the slit (9) or pore may be suitably determined taking into consideration the advantage of air supply and the disadvantage of the exudate spreading outside.

Providing another sheet material which covers and hides the slit (9) or pore without blocking them is effective in preventing the exudate from soiling clothes and bedding.

In the first embodiment and each Modified Example, the protective layer (4) is fixed to the absorbing retaining layer (3) with an adhesive which is not shown in the figures. In the present invention, however, the protective layer (4) may be integrally fixed to the absorbing retaining layer (3) with the adhesive area (7) extending inwardly beyond the edges of the peripheral portion (6) on the surface of the protective layer (4).

### Production Process

The process for producing the wound dressing (5) is not limited to a particular production method as long as each of the above layers can be integrally stacked and the objects of the present invention is not hampered, and a publicly known production method may be suitably adopted. Each layer may be integrally stacked at the same time; or after particular layers are stacked and joined, another layer may be stacked on and integrated with the laminated body.

However, in the production of the wound dressing (5) in which the liquid-permeable layer (1) is stacked on and integrated with the liquid-permeation restricting layer (2) or in which the liquid-permeation restricting layer (2) is stacked on and integrated with the absorbing retaining layer (3), a preferred step is to partially apply a hot-melt adhesive to one surface which is to face another layer, and stack the another layer on the applied surface to join together.

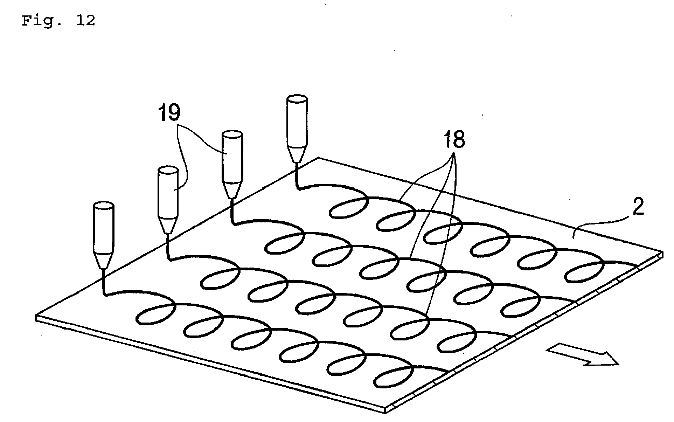

In particular, for example, in cases where the liquid-permeable layer (1) is stacked on the liquid-permeation restricting layer (2), a hot-melt adhesive (18) is partially applied to the liquid-permeation restricting layer (2) as shown in, for example, Fig. 12, and the liquid-permeable layer (1) is stacked on the applied surface to join the two layers (1, 2) together.

When the adhesive (18) is partially applied as described above, the part on which the adhesive (18) has been applied prevents the migration of the exudate between the two layers, while the part on which the adhesive (18) has not been applied allows the migration of the exudate from one layer to another layer.

The application pattern with which the adhesive (18) is partially applied is not particularly limited, and various types of application patterns may be adopted. Preferred application patterns are patterns in which an applied part and a non-applied part alternately appear, for example, a dot pattern, a striped pattern, a lattice pattern, or the like. Particularly preferred application pattern is a spiral pattern as shown in, for example, Fig. 12. Such spiral pattern is easily realizable by discharging a hot-melt adhesive (18) from a discharge nozzle (19) from above the sheet material while the sheet material is fed, and thus excellent productivity and good joint condition can be achieved.

The wound dressing (5) of the present invention produced as described above can retain the exudate (16) at the place where it oozes from a wound site (15) to prevent the exudate (16) from spreading over a larger area while maintaining a moist environment by not sucking up the exudate (16) rapidly. By retaining the exudate (16) in the area around the wound site (15), the therapeutic effect can be enhanced, while unnecessary spread of the exudate (16), which causes irritation of unwounded normal skin, can be prevented.

### Second Embodiment

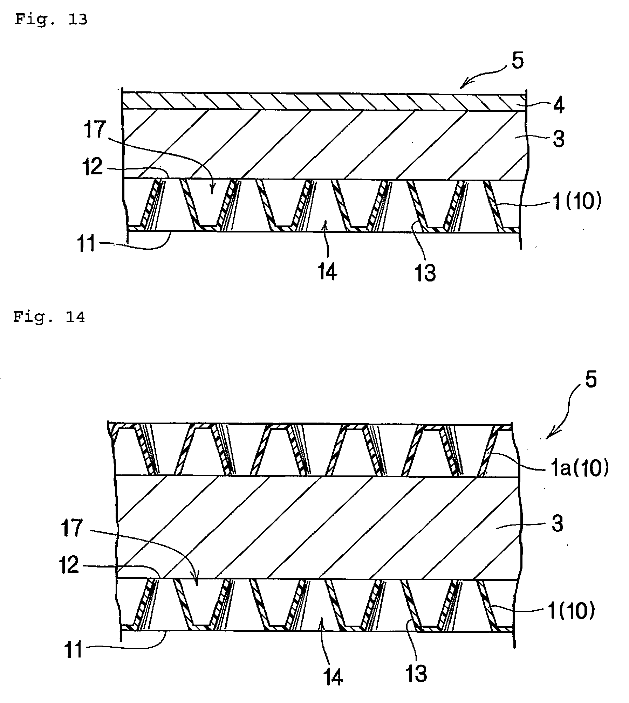

The above first embodiment has described the case where the liquid-permeation restricting layer (2) is provided between the liquid-permeable layer (1) and the absorbing retaining layer (3). In the present invention, however, the liquid-permeation restricting layer may be omitted as in, for example, a second embodiment shown in Fig. 13.

That is, unlike the above first embodiment, in the second embodiment, the liquid-permeation restricting layer is omitted and the absorbing retaining layer (3) is directly stacked on the second surface (12) of the liquid-permeable layer (1). This second embodiment is preferable in that the omission of the liquid-permeation restricting layer simplifies the production and reduces the production cost. Although the liquid-permeation restricting layer is omitted in the second embodiment, the similar effect to that of the wound dressing having the liquid-permeation restricting layer can be exerted by utilizing a highly hydrophobic material for the liquid-permeable layer (1), preferably, for example, a highly hydrophobic material having a contact angle with physiological saline of 85 degrees or more.

### Third Embodiment

The wound dressing (5) of the present invention as in, for example, a third embodiment shown in Fig. 14 may have the structure in which the following layers are stacked and integrated together in the following order from the side to face a wound site: the liquid-permeable layer (1), the absorbing retaining layer (3), and a second liquid-permeable layer (1a). In this case, the material of the second liquid-permeable layer (1a) can be selected from the above-described materials used for the above liquid-permeable layer. Each of the liquid-permeable layers (1, 1a) may be made of an identical material or different materials.

The wound dressing (5) of the third embodiment is provided with the second liquid-permeable layer (1a) on the opposite surface of the absorbing retaining layer (3) to the surface which is closer to a wound site. Hence, the wound dressing (5) has a better breathability, which is advantageous for preventing sweating and damping of the skin other than the wound site (15). Such a wound dressing is especially suitable for the treatment of impetigo. Especially when both liquid-permeable layers (1, 1a) are made of an identical material in the third embodiment, any of the liquid-permeable layers may be in contact with a wound site. Thus no attention is needed for which side is the front or the back of the wound dressing (5), which is advantageous in facilitating the application to the patient.

In this embodiment, the liquid-permeable layers (1, 1a) are directly joined to the absorbing retaining layer (3). In the present invention, however, the liquid-permeation restricting layer similar to that used in the first embodiment may be provided between either of the liquid-permeable layers (1, 1a) and the absorbing retaining layer (3) or between each of the liquid-permeable layers (1, 1a) and the absorbing retaining layer (3).

### Fourth Embodiment

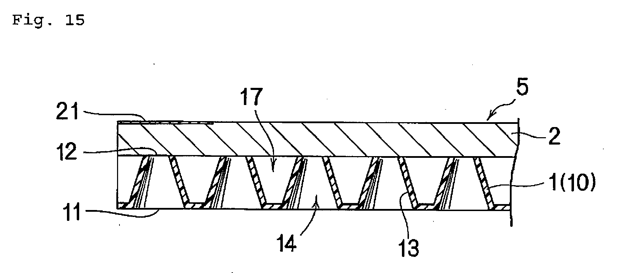

Fig. 15 shows a fourth embodiment of the wound dressing (5) of the present invention.

Unlike the above first embodiment, in the fourth embodiment the absorbing retaining layer (3) and the protective layer (4) are omitted, and the following layers are stacked and integrated together in the following order from the side to be used in contact with a wound site: the liquid-permeable layer (1) and the liquid-permeation restricting layer (2).

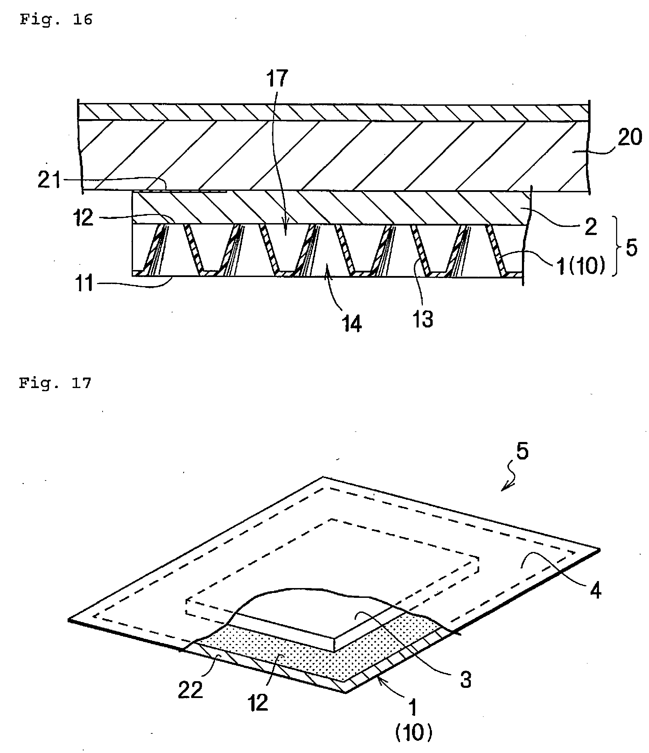

The wound dressing (5) of the fourth embodiment is used by applying its liquid-permeation restricting layer (2) to the absorbing surface of an absorbent article (20) such as a paper diaper, as shown in, for example, Fig. 16. For example, a patient who has been bedridden for a long period of time etc. may develop a bed sore. In this case, wearing a paper diaper (20) to which the wound dressing (5) of the fourth embodiment is attached is preferable in that the wound site (15) can be easily protected by the wound dressing (5).

With respect to the above, the liquid-permeation restricting layer (2) preferably has an adhesive layer (21) on the opposite surface to the surface which is closer to the wound site, as shown in Figs. 15 and 16. Examples of the adhesive used for the adhesive layer (21) include an acrylic adhesive, a natural rubber adhesive, a synthetic rubber adhesive, a silicone adhesive, a vinyl ether adhesive, and a polyester adhesive. Preferred is an acrylic adhesive in view of the stability of the quality of the adhesive, the controllability of the adhesion properties, the long term stability of the adhesion properties, non-irritability to the skin, and the like. The acrylic adhesive is preferably a copolymer which is obtainable by copolymerizing a (meth)acrylic acid alkyl ester as a principal monomer with a copolymerizable monomer.

In cases where the adhesive layer is not provided, a surgical tape or the like can be used to fix the wound dressing to the absorbent article (20).

Also in the fourth embodiment described above, in cases where the liquid-permeable layer (1) has excellent hydrophobicity, the liquid-permeation restricting layer (2) can be omitted as in the second embodiment. This embodiment will cost lower. However, providing the liquid-permeation restricting layer (2) as in the fourth embodiment is preferable in that it will prevent the excessive absorption of the exudate, give the wound dressing (5) stiffness (what is called "sturdiness"), and make the wound dressing (5) easy to handle.

The above structure having the adhesive layer (21) on the opposite surface to the surface which is closer to the wound site is not limited to the one in the fourth embodiment and, in the present invention, can be applied to, for example, the wound dressing having the absorbing retaining layer (3) as in the first, second, and third embodiments. For example, in cases where an amount of exudate oozed from a wound site is excessive compared to the absorption performance of the absorbing retaining layer (3), preferably an absorbent article such as a paper diaper is attached via the adhesive layer (21) to the opposite surface of the wound dressing to the surface which is to face the wound site for sufficient absorption of the exudate.

In addition, the article to be attached via the adhesive layer (21) is not limited to absorbent articles such as a paper diaper. Especially in cases where the amount of the exudate from the wound site is smaller, for example, in the case of sunburn or the like, the article may be clothes or the like, such as underwear directly in contact with the patient's skin. In this case, omission of the absorbing retaining layer as in the fourth embodiment or the use of a thin absorbing retaining layer is preferable in that the whole wound dressing protecting the wound site can be made thinner and that such a thin wound dressing has no risk of restraining the patient's movement.

### Fifth Embodiment

Fig. 17 shows a fifth embodiment of the wound dressing (5) of the present invention.

Unlike the above first embodiment, the absorbing retaining layer (3) is not integrated with the other layers in the fifth embodiment. That is, in the wound dressing (5) of the fifth embodiment, the circumferential part (22) on the second surface (12) of the liquid-permeable layer (1) formed of the surface sheet (10) is integrated with the protective layer (4) by welding or the like to form a bag. Between the liquid-permeable layer (1) and the protective layer (4), the absorbing retaining layer (3) is inserted without being fixed to both layers (1, 4).

The structure in the fifth embodiment in which the absorbing retaining layer (3) is not fixed to the liquid-permeable layer (1) or the protective layer (4) with an adhesive or the like is preferable in that active absorption by the absorbing retaining layer (3) hardly occurs, which results in restriction on the migration of the exudate from the liquid-permeable layer (1) to the absorbing retaining layer (3) and excellent retention of the exudate between the wound site and the liquid-permeable layer (1). In addition, since the absorbing retaining layer (3) between the liquid-permeable layer (1) and the protective layer (4) is movable along the second surface (12) of the liquid-permeable layer (1), even when the shear stress acts on part of the wound dressing (5), for example, on the absorbing retaining layer (3), the movement of the absorbing retaining layer (3) can absorb the shear stress at the wound site and alleviate the stress applied on the wound site. Consequently, the liquid-permeable layer (1) formed of the surface sheet (10) hardly causes a relative position gap between the wound dressing and the wound site, which is highly suitable for the treatment and prevention of a bed sore. Further, since the absorbing retaining layer (3) is relatively displaceable from the liquid-permeable layer (1) and the protective layer (4), the wound dressing (5) is totally flexible and excellent in texture.

In the fifth embodiment, the absorbing retaining layer (3) is disposed between the liquid-permeable layer (1) and the protective layer (4). In the present invention, however, the liquid-permeation restricting layer (2) may be integrally stacked on at least any one of the second surface (12) of the liquid-permeable layer (1) and the surface of the absorbing retaining layer (3) facing the second surface of the liquid-permeable layer. In the fifth embodiment, as long as not fixed to the liquid-permeable layer (1), the absorbing retaining layer (3) may be fixed to the protective layer (4). This structure is preferable in that the migration of the exudate from the liquid-permeable layer (1) to the absorbing retaining layer (3) can be restricted and that the absorbing retaining layer (3) can be held to a predetermined position relative to the wound site.

### EXAMPLES

The surface sheet of the present invention used for the liquid-permeable layer will be described in more detail below with reference to Examples, but the present invention is not limited thereto.

### Examples 1 to 3 and Comparative Examples 1 and 2

As the resin sheet material forming the surface sheet, a polyethylene sheet material was used in Example 1, a polyvinyl chloride sheet material was used in Example 2, a polyvinyl alcohol sheet material was used in Example 3, a nylon 6 sheet material was used in Comparative Example 1, and a polyethylene sheet material surface-treated with an oil containing a surfactant was used in Comparative Example 2. The "polyethylene sheet material surface-treated with an oil containing a surfactant" in Comparative Example 2 was, in particular, one which is commonly used as a surface material of a marketed sanitary napkin.

Each of the predetermined resin sheet materials of Examples and Comparative Examples was made into a mesh sheet material having a distance between the first surface and the second surface (thickness) of 480 µm, by forming a large number of penetration pores therein. An airlaid nonwoven fabric made of a pulp and a polyolefin binder fiber was stacked on and integrated with the second surface of the mesh sheet material and the obtained sheet material was used as a sample. The integration of the mesh sheet material and the airlaid nonwoven fabric was performed by applying 3 g/m² of a synthetic rubber hot-melt adhesive in a dot pattern.

The penetration pores were tapered pores having the pore opening diameter on the first surface of 615 µm, which was 1.25 times larger than that on the second surface. The pore opening density was 200 pores/cm², the pore opening ratio of the entire sheet material was 42%, and the capacity of the retaining space per penetration pore was 0.1 µL.

The contact angles of the samples of Examples 1 to 3 and Comparative Examples 1 and 2 with physiological saline were measured according to JIS K 2396. The measurement results are shown in Table 1.

**Table 1**

| Sample | | | Absorbing retaining layer |
|---|---|---|---|
| | Liquid-permeable layer | contact angle (degree) | |
| Example 1 | Polyethylene sheet material provided with penetration pores | 110 | Airlaid nonwoven |
| Example 2 | Polyvinyl chloride sheet material provided with penetration pores | 95 | Airlaid nonwoven |
| Example 3 | Polyvinyl alcohol sheet material provided with penetration pores | 85 | Airlaid nonwoven |
| Comparative Example 1 | Nylon 6 sheet material provided with penetration pores | 82 | Airlaid nonwoven |
| Comparative Example 2 | Polyethylene sheet material provided with penetration pores and treated with surfactant | 68 | Airlaid nonwoven |

### Comparative Experiment

One opening of a cylinder tube 50 mm in inner diameter was made in contact with the surface of each sample of Examples 1 to 3 and Comparative Examples 1 and 2, and 10 mL of physiological saline was gently poured through the other opening of the cylinder tube to be absorbed by the sample. After the pouring of the physiological saline, the sample and the tube were left to stand for 10 minutes. The cylinder tube was then removed and a filter paper was placed on the part where the physiological saline was absorbed. A 2.5-kgf load 90 mm in diameter was applied on the filter paper and left to stand for 2 minutes so that the filter paper absorbed the moisture which remained on the surface of the sample, i.e., in the retaining space of the mesh sheet and on the surface of the mesh sheet. Next, by measuring the weight increase in the filter paper, the amount of the moisture absorbed in the filter paper, i.e., the moisture which remained in the retaining space of the mesh sheet and on the surface of the mesh sheet, was determined. In this way, the amount of the residual moisture per area was calculated. The measurement results are shown in Table 2. The physiological saline used for the measurement in Comparative Experiment and for the measurement of the contact angles was prepared by dissolving, in 1 L of deionized water, sodium chloride (NaCl) at a concentration of 8. 30 g/L (sodium ion: 142 mmol) and calcium chloride dihydrate (CaCl₂·2H₂O) at a concentration of 0.37 g/L (calcium ion: 2.5 mmol).

**Table 2**

| Sample | Weight increase in filter paper (g) | Residual moisture (µL/cm²) |
|---|---|---|
| Example 1 | 0.28 | 14 |
| Example 2 | 0.24 | 12 |
| Example 3 | 0.20 | 10 |
| Comparative Example 1 | 0.10 | 5 |
| Comparative Example 2 | 0.02 | 1 |

The above measurement results revealed that the mesh sheets in Comparative Examples 1 and 2 failed to retain a sufficient amount of moisture and that the mesh sheets of the present invention in Examples 1 to 3 retained a sufficient amount of moisture. Therefore, wound dressings provided with the sheets in Comparative Examples 1 and 2 may cause dryness on the wound site and may fail to maintain a moist environment on the wound site. On the other hand, wound dressings provided with the sheets of the present invention in Examples 1 to 3 can maintain a moist environment on the wound site and has no risk of sticking tight to the wound site.

The wound dressings explained in the above embodiments and Modified Examples are only examples for demonstrating the technical idea of the present invention. The material, structure of lamination, shape, size, usage, and the like are not limited to the embodiments or Modified Examples, and can be variously modified within the scope of the claims of the present invention.

For example, in cases where each layer is a thin layer, a reinforcement layer may be provided to facilitate handling of the wound dressing. For the purpose of maintaining cleanliness of the surface to be in contact with a wound site or protecting the adhesive layer, a release layer formed of a release paper may be provided on the outer surface of the wound dressing. These reinforcement layer and release layer may be removed at the time of applying the wound dressing to a wound site.

In the above embodiments, the second surface of the liquid-permeable layer is formed to have concavities and convexities. In the present invention, however, the second surface may be formed in a smooth surface. The above first embodiment and Modified Examples have described the case where the adhesive area is formed on the peripheral portion of the protective layer. In the present invention, however, the adhesive area may be omitted and the wound dressing may be fixed to a wound site with an adhesive plaster or the like. Needless to say, the penetration pores are not limited to tapered pores.

The above embodiments have described the case where the wound dressing is used as a usual wound dressing. However, what is needed for the wound dressing and the surface sheet of the present invention is only that the first surface of the surface sheet faces a wound site during use, and the usage of the wound dressing and the surface sheet is not limited to particular one.

For example, the wound dressing and the surface sheet of the present invention can be used in what is called negative pressure wound therapy, in which a dressing material is used to cover a wound site to form a closed area and negative pressure is applied to the closed area with a suction means. In negative pressure wound therapy, a screen means, for example, a polyurethane foam or the like is disposed between the wound site and the suction means to prevent granulation tissue on the wound site from being sucked. In this case, the wound dressing and the surface sheet of the present invention can be disposed on one side of the screen means which side is to face a wound site so that the first surface of the surface sheet faces the wound site. Further, the wound dressing and the surface sheet of the present invention can also be used as the screen means. In any case, the wound dressing and the surface sheet of the present invention is preferable in that a moderate amount of exudate from a wound site is retained between the wound site and the surface sheet, which prevents excessive suction of the exudate by the suction means, and that the exudate which has passed through the penetration pores of the surface sheet is prevented from flowing back to the wound site.

### INDUSTRIAL APPLICABILITY

A wound dressing utilizing the surface sheet of the present invention can retain exudate to prevent it from spreading over a larger area while maintaining a moist environment, can be easily peeled off after use, can prevent skin redness, heat rash, or offensive smell, and can fit various shapes of wound surfaces. Therefore such a wound dressing is suitable for the treatment of various kinds of wounds and most suitable for the prevention and treatment of a bed sore in particular.

### REFERENCE SIGNS LIST

1. Liquid-permeable layer
1a. Second liquid-permeable layer
2. Liquid-permeation restricting layer
3. Absorbing retaining layer
4. Protective layer
5. Wound dressing
6. Peripheral portion of protective layer (4)
7. Adhesive area
8. Non-adhesive area
9. Slit
10. Surface sheet
11. First surface
12. Second surface
13. Penetration pore
14. Retaining space
15. Wound site
16. Exudate
17. Concavity
18. Hot-melt adhesive
19. Discharge nozzle
20. Absorbent article (paper diaper)
21. Adhesive layer
22. Circumferential part of liquid-permeable layer (1)

## Claims

1. A surface sheet for a wound dressing,
the surface sheet being disposed on at least a portion of the wound dressing (5) which portion is to face a wound site and
comprising a resin sheet material having a first surface (11) which is to face the wound site (15), a second surface (12) which is the opposite surface to the first surface, and a large number of penetration pores (13) penetrating through the sheet material from the first surface (11) to the second surface (12) in the thickness direction,
the penetration pores (13) allowing the permeation of a liquid from the first surface (11) to the second surface (12), and the first surface (11) being hydrophobic, and wherein the opening area of the penetration pores (13) being a circle between 280 and 1400 µm in diameter, the density of the penetration pores (13) being between 50 and 400 pores/cm², the pore opening diameter ratio of the circle of the first surface (11) to the second surface (12) being between 1.1 and 1.8, the depth of the penetration pores (13) being between 100 and 2000 µm, the capacity of the retaining space formed within the penetration pores (13) being between 0.015 and 0.55 micro-liters, and
the sheet material comprises a low-density polyethylene resin material.

2. The surface sheet for a wound dressing according to claim 1, wherein the first surface (11) has a contact angle with physiological saline of 85 degrees or more.

3. The surface sheet for a wound dressing according to claim 1 or 2, wherein the first surface (11) has a surface tension of 40 dyne/cm or less.

4. The surface sheet for a wound dressing according to any of claims 1 to 3, wherein the first surface (11) is coated with one or more water-repellent materials selected from the group consisting of silicone, polyurethane, a styrene-butadiene-styrene block copolymer, a tetrafluoroethylene-hexafluoropropylene copolymer, a tetrafluoroethylene-perfluoroalkylvinyl ether copolymer, and polytetrafluoroethylene.

5. A wound dressing comprising at least two layers, a liquid-permeable layer (1) and an absorbing retaining layer (3),
the two layers being stacked in the following order from the side to be used in contact with a wound site (15): the liquid-permeable layer (1) and the absorbing retaining layer (3),
the liquid-permeable layer (1) comprising the surface sheet (10) according to any of claims 1 to 4, and
the absorbing retaining layer (3) comprising a sheet material capable of absorbing and retaining water.

6. The wound dressing according to claim 5, wherein a second liquid-permeable layer (1a) is further stacked on the opposite surface of the absorbing retaining layer (3) to the surface on which the liquid-permeable layer (1) is disposed.

7. The wound dressing according to claim 5, wherein a protective layer (4) is further provided on the opposite surface of the absorbing retaining layer (3) to the surface which is closer to the wound site, and the protective layer (4) comprises a resin film, a woven fabric, a knitted fabric, or a nonwoven fabric.

8. The wound dressing according to claim 7, wherein the protective layer (4), which has a wider area than those of the other layers, covers all of the other layers and extends outward beyond the edges of the other layers to form a peripheral portion (6), and the peripheral portion (6) has an adhesive area (7) on at least a part of the surface on which the other layers are stacked.

9. The wound dressing according to claim 8, wherein the peripheral portion (6) of the protective layer (4) has a non-adhesive area (8), which is other than the adhesive area (7).

10. The wound dressing according to claim 8, wherein a part of the peripheral portion (6) of the protective layer (4) outside the edges of the other layers is omitted.

11. The wound dressing according to claim 8, wherein the peripheral portion (6) of the protective layer (4) has a slit (9) or a small pore along the perimeters of the other layers.

12. The wound dressing according to any of claims 5 to 11, wherein a liquid-permeation restricting layer (2) is disposed between the liquid-permeable layer (1, 1a) and the absorbing retaining layer (3),
the liquid-permeation restricting layer (2) comprises a microporous film, a woven fabric, a knitted fabric, or a nonwoven fabric, each of which comprises a hydrophobic material, and
through the liquid-permeation restricting layer (2), a liquid is allowed to migrate from the liquid-permeable layer (1) to the absorbing retaining layer (3).

13. The wound dressing according to claim 12, wherein the liquid-permeation restricting layer (2) comprises a polyolefin resin and has a permeability measured according to JIS L 1096 of 5 to 2000 cm³/cm²·S and a water repellency measured according to JIS L 1092 of grade 3 or higher.

14. The wound dressing according to claim 12 or 13, wherein the liquid-permeation restricting layer (2) comprises a nonwoven fabric comprising a polypropylene fiber.

15. The wound dressing according to any of claims 5 to 14, wherein the absorbing retaining layer (3) comprises an airlaid nonwoven fabric.

16. The wound dressing according to any of claims 5 to 15, wherein the absorbing retaining layer (3) comprises fluff pulp.

17. The wound dressing according to claim 16, wherein the absorbing retaining layer (3) further comprises a super absorbent polymer, and the weight ratio of the super absorbent polymer and the fluff pulp is 10:90 to 25:75.

18. The wound dressing according to claim 17, wherein the super absorbent polymer is a sodium polyacrylate polymer.

19. The wound dressing according to any of claims 5 to 18, wherein the absorbing retaining layer (3) is partially not joined to an adjacent layer which is on the side closer to the wound site.

20. The wound dressing according to claim 19, wherein the absorbing retaining layer (3) is not integrated with the liquid-permeable layer (1) and is movable along the second surface (12) of the liquid-permeable layer (1).

21. A wound dressing comprising a liquid-permeable layer (1) comprising the surface sheet (10) according to any of claims 1 to 4.

22. The wound dressing according to claim 21, wherein a liquid-permeation restricting layer (2) is stacked on the second surface (12) of the surface sheet (10),
the liquid-permeation restricting layer (2) comprises a microporous film, a woven fabric, a knitted fabric, or a nonwoven fabric, each of which comprises a hydrophobic material, and
the liquid-permeation restricting layer (2) allows the permeation of a liquid in the thickness direction.

23. The wound dressing according to claim 22, wherein the liquid-permeation restricting layer (2) comprises a polyolefin resin and has a permeability measured according to JIS L 1096 of 5 to 2000 cm³/cm²·S and a water repellency measured according to JIS L 1092 of grade 3 or higher.

24. The wound dressing according to claim 22 or 23, wherein the liquid-permeation restricting layer (2) comprises a nonwoven fabric comprising a polypropylene fiber.

25. The wound dressing according to any of claims 5 to 24, comprising an adhesive layer (21) on the opposite surface to the surface which is to face the wound site (15).

## Patentansprüche

1. Oberflächenfolie für einen Wundverband,
wobei die Oberflächenfolie an zumindest einem Abschnitt des Wundverbandes (5) angeordnet ist, wobei der Abschnitt der Wundstelle zugewandt sein soll, und
ein Harz-Folienmaterial mit einer ersten Oberfläche (11), die der Wundstelle (15) zugewandt sein soll, einer zweiten Oberfläche (12), die die der ersten Oberfläche entgegengesetzte Oberfläche ist, und eine große Anzahl von Durchgangsporen (13), die das Folienmaterial von der ersten Oberfläche (11) zur zweiten Oberfläche (12) in der Dickenrichtung durchqueren, umfasst, wobei die Durchgangsporen (13) das Durchdringen einer Flüssigkeit von der ersten Oberfläche (11) zur zweiten Oberfläche (12) ermöglichen und die erste Oberfläche (11) hydrophob ist, und wobei die Öffnungsfläche der Durchgangsporen (13) ein Kreis mit einem Durchmesser zwischen 280 und 1400 µm ist, die Dichte der Durchgangsporen (13) zwischen 50 und 400 Poren/cm² liegt, das Verhältnis des Porenöffnungsdurchmessers des Kreises der ersten Oberfläche (11) zur zweiten Oberfläche (12) zwischen 1,1 und 1,8 liegt, die Tiefe der Durchgangsporen (13) zwischen 100 und 2000 µm liegt, die Kapazität des in den Durchgangsporen (13) gebildeten Rückhalteraums zwischen 0,015 und 0,55 Mikrolitern liegt und das Folienmaterial ein Harzmaterial aus Polyethylen niederer Dichte umfasst.

2. Oberflächenfolie für einen Wundverband nach Anspruch 1, wobei die erste Oberfläche (11) einen Kontaktwinkel mit physiologischer Kochsalzlösung von 85 Grad oder mehr aufweist.

3. Oberflächenfolie für einen Wundverband nach Anspruch 1 oder 2, wobei die erste Oberfläche (11) eine Oberflächenspannung von 40 Dyn/cm oder weniger aufweist.

4. Oberflächenfolie für einen Wundverband nach einem der Ansprüche 1 bis 3, wobei die erste Oberfläche (11) mit einem oder mehreren wasserabweisenden Materialien, ausgewählt aus der Gruppe, bestehend aus Silikon, Polyurethan, einem Styrol-Butadien-Styrol-Blockcopolymer, einem Tetrafluorethylen-Hexafluorpropylen-Copolymer, einem Tetrafluorethylen-Perfluoralkylvinylether-Copolymer und Polytetrafluorethylen, beschichtet ist.

5. Wundverband, umfassend zumindest zwei Schichten, eine flüssigkeitsdurchlässige Schicht (1) und eine absorbierende Rückhalteschicht (3), wobei die beiden Schichten in der folgenden Reihenfolge ausgehend von der die Wundstelle (15) kontaktierenden Seite geschichtet sind: die flüssigkeitsdurchlässige Schicht (1) und die absorbierende Rückhalteschicht (3),
wobei die flüssigkeitsdurchlässige Schicht (1) die Oberflächenfolie (10) nach einem der Ansprüche 1 bis 4 umfasst und
die absorbierende Rückhalteschicht (3) ein Folienmaterial umfasst, das Wasser absorbieren und zurückhalten kann.

6. Wundverband nach Anspruch 5, wobei ferner eine zweite flüssigkeitsdurchlässige Schicht (1a) auf die Oberfläche der absorbierenden Rückhalteschicht (3) geschichtet ist, die der Oberfläche, auf der die flüssigkeitsdurchlässige Schicht (1) angeordnet ist, entgegengesetzt ist.

7. Wundverband nach Anspruch 5, wobei ferner eine Schutzschicht (4) auf der Oberfläche der absorbierenden Rückhalteschicht (3) vorgesehen ist, die der Oberfläche, die näher an der Wundstelle liegt, entgegensetzt ist, und die Schutzschicht (4) einen Harzfilm, ein Gewebe, ein Gewirk oder ein Faservlies umfasst.

8. Wundverband nach Anspruch 7, wobei die Schutzschicht (4), die eine breitere Fläche als die der anderen Schichten hat, alle anderen Schichten bedeckt und sich nach außen über die Ränder der anderen Schichten hinaus erstreckt und so einen Umfangsabschnitt (6) bildet, und der Umfangsabschnitt (6) eine Haftfläche (7) auf zumindest einem Teil der Oberfläche, auf die die anderen Schichten geschichtet sind, aufweist.

9. Wundverband nach Anspruch 8, wobei der Umfangsabschnitt (6) der Schutzschicht (4) eine nicht haftende Fläche (8) aufweist, die von der Haftfläche (7) verschieden ist.

10. Wundverband nach Anspruch 8, wobei ein Teil des Umfangsabschnitts (6) der Schutzschicht (4) außerhalb der Ränder der anderen Schichten weggelassen ist.

11. Wundverband nach Anspruch 8, wobei der Umfangsabschnitt (6) der Schutzschicht (4) einen Schlitz (9) oder eine kleine Pore entlang den Umfängen der anderen Schichten aufweist.

12. Wundverband nach einem der Ansprüche 5 bis 11, wobei eine den Flüssigkeitsdurchlass einschränkende Schicht (2) zwischen der flüssigkeitsdurchlässigen Schicht (1, 1a) und der absorbierenden Rückhalteschicht (3) angeordnet ist,
die den Flüssigkeitsdurchlass einschränkende Schicht (2) einen mikroporösen Film, ein Gewebe, ein Gewirk oder ein Faservlies umfasst, die jeweils ein hydrophobes Material umfassen, und
durch die den Flüssigkeitsdurchlass einschränkende Schicht (2) eine Flüssigkeit von der flüssigkeitsdurchlässigen Schicht (1) zu der absorbierenden Rückhalteschicht (3) migrieren kann.

13. Wundverband nach Anspruch 12, wobei die den Flüssigkeitsdurchlass einschränkende Schicht (2) ein Polyolefinharz umfasst und eine Durchlässigkeit, gemessen nach JIS L 1096, von 5 bis 2000 cm³/cm²·S und ein Wasserabweisungsvermögen, gemessen nach JIS L 1092, von Stufe 3 oder höher aufweist.

14. Wundverband nach Anspruch 12 oder 13, wobei die den Flüssigkeitsdurchlass einschränkende Schicht (2) ein Faservlies, umfassend eine Polypropylenfaser, umfasst.

15. Wundverband nach einem der Ansprüche 5 bis 14, wobei die absorbierende Rückhalteschicht (3) ein Airlaid-Faservlies umfasst.

16. Wundverband nach einem der Ansprüche 5 bis 15, wobei die absorbierende Rückhalteschicht (3) Flockenzellstoff umfasst.

17. Wundverband nach Anspruch 16, wobei die absorbierende Rückhalteschicht (3) ferner ein superabsorbierendes Polymer umfasst und das Gewichtsverhältnis des superabsorbierenden Polymers und des Flockenzellstoffs 10 : 90 bis 25 : 75 beträgt.

18. Wundverband nach Anspruch 17, wobei das superabsorbierende Polymer ein Natriumpolyacrylatpolymer ist.

19. Wundverband nach einem der Ansprüche 5 bis 18, wobei die absorbierende Rückhalteschicht (3) teilweise nicht mit einer benachbarten Schicht, die sich auf der der Wundstelle näherliegenden Seite befindet, verbunden ist.

20. Wundverband nach Anspruch 19, wobei die absorbierende Rückhalteschicht (3) nicht in die flüssigkeitsdurchlässige Schicht (1) integriert und entlang der zweiten Oberfläche (12) der flüssigkeitsdurchlässigen Schicht (1) beweglich ist.

21. Wundverband, umfassend eine flüssigkeitsdurchlässige Schicht (1), umfassend die Oberflächenfolie (10) nach einem der Ansprüche 1 bis 4.

22. Wundverband nach Anspruch 21, wobei
eine den Flüssigkeitsdurchlass einschränkende Schicht (2) auf die zweite Oberfläche (12) der Oberflächenfolie (10) geschichtet ist,
die den Flüssigkeitsdurchlass einschränkende Schicht (2) einen mikroporösen Film, ein Gewebe, ein Gewirk oder ein Faservlies umfasst, die jeweils ein hydrophobes Material umfassen, und
die den Flüssigkeitsdurchlass einschränkende Schicht (2) das Durchdringen einer Flüssigkeit in der Dickenrichtung ermöglicht.

23. Wundverband nach Anspruch 22, wobei die den Flüssigkeitsdurchlass einschränkende Schicht (2) ein Polyolefinharz umfasst und eine Durchlässigkeit, gemessen nach JIS L 1096, von 5 bis 2000 cm³/cm²·S und ein Wasserabweisungsvermögen, gemessen nach JIS L 1092, von Stufe 3 oder höher aufweist.

24. Wundverband nach Anspruch 22 oder 23, wobei die den Flüssigkeitsdurchlass einschränkende Schicht (2) ein Faservlies, umfassend eine Polypropylenfaser, umfasst.

25. Wundverband nach einem der Ansprüche 5 bis 24, umfassend eine Haftschicht (21) auf der der Oberfläche, die der Wundstelle (15) zugewandt sein soll, entgegengesetzten Oberfläche.

## Revendications

1. Feuille de surface pour un pansement pour plaies,
la feuille de surface étant disposée sur au moins une portion du pansement pour plaies (5), laquelle portion doit faire face à un site de la plaie et
comprenant un matériau de feuille de résine ayant une première surface (11) qui doit faire face au site de la plaie (15), une seconde surface (12) qui est la surface opposée à la première surface, et un nombre important de pores de pénétration (13) pénétrant à travers le matériau de feuille de la première surface (11) à la seconde surface (12) dans le sens de l'épaisseur, les pores de pénétration (13) permettant l'infiltration d'un liquide de la première surface (11) vers la seconde surface (12), et la première surface (11) étant hydrophobe, et
dans laquelle la zone d'ouverture des pores de pénétration (13) est un cercle d'un diamètre compris entre 280 et 1400 µm, la densité des pores de pénétration (13) est comprise entre 50 et 400 pores/cm², le rapport du diamètre d'ouverture des pores du cercle de la première surface (11) sur la seconde surface (12) est compris entre 1,1 et 1,8, la profondeur des pores de pénétration (13) est comprise entre 100 et 2000 µm, la capacité de l'espace de rétention formé à l'intérieur des pores de pénétration (13) est comprise entre 0,015 et 0,55 microlitres, et
le matériau de feuille comprend un matériau de résine polyéthylène faible densité.

2. Feuille de surface pour un pansement pour plaies selon la revendication 1, dans laquelle la première surface (11) a un angle de contact avec du sérum physiologique supérieur ou égal à 85 degrés.

3. Feuille de surface pour un pansement pour plaies selon la revendication 1 ou 2, dans laquelle la première surface (11) a une tension superficielle inférieure ou égale à 40 dynes/cm.

4. Feuille de surface pour un pansement pour plaies selon l'une quelconque des revendications 1 à 3, dans laquelle la première surface (11) est revêtue d'un ou plusieurs matériaux hydrofuges choisis dans le groupe constitué de silicone, polyuréthane, copolymère séquencé styrène-butadiène-styrène, copolymère de tétrafluoroéthylènehexafluoropropylène, copolymère de tétrafluoroéthylène-éther perfluoroalkylvinylique et polytétrafluoroéthylène.

5. Pansement pour plaies comprenant au moins deux couches, une couche perméable au liquide (1) et une couche de rétention absorbante (3),
les deux couches étant empilées dans l'ordre suivant en partant du côté devant être utilisé en contact avec un site de plaie (15) : la couche perméable au liquide (1) et la couche de rétention absorbante (3),
la couche perméable au liquide (1) comprenant la feuille de surface (10) selon l'une quelconque des revendications 1 à 4, et
la couche de rétention absorbante (3) comprenant un matériau de feuille capable d'absorber et de retenir l'eau.

6. Pansement pour plaies selon la revendication 5, dans lequel une seconde couche perméable au liquide (1a) est en outre empilée sur la surface de la couche de rétention absorbante (3) opposée à la surface sur laquelle est disposée la couche perméable au liquide (1).

7. Pansement pour plaies selon la revendication 5, dans lequel une couche protectrice (4) est en outre placée sur la surface de la couche de rétention absorbante (3) opposée à la surface qui est la plus proche du site de la plaie, et la couche protectrice (4) comprend un film de résine, un tissu tissé, un tissu maillé ou un tissu non tissé.

8. Pansement pour plaies selon la revendication 7, dans lequel la couche protectrice (4), qui a une surface plus large que celle des autres couches, recouvre toutes les autres couches et s'étend vers l'extérieur au-delà des bords des autres couches pour former une portion périphérique (6), et la portion périphérique (6) a une surface adhésive (7) sur au moins une partie de la surface sur laquelle sont empilées les autres couches.

9. Pansement pour plaies selon la revendication 8, dans lequel la portion périphérique (6) de la couche protectrice (4) a une surface non adhésive (8), qui est différente de la surface adhésive (7).

10. Pansement pour plaies selon la revendication 8, dans lequel une partie de la portion périphérique (6) de la couche protectrice (4) à l'extérieur des bords des autres couches est omise.

11. Pansement pour plaies selon la revendication 8, dans lequel la portion périphérique (6) de la couche protectrice (4) comporte une fente (9) ou un petit pore le long des périmètres des autres couches.

12. Pansement pour plaies selon l'une quelconque des revendications 5 à 11, dans lequel une couche limitant l'infiltration de liquide (2) est disposée entre la couche perméable au liquide (1, 1a) et la couche de rétention absorbante (3),
la couche limitant l'infiltration de liquide (2) comprend un film microporeux, un tissu tissé, un tissu maillé ou un tissu non tissé, chacun comprenant un matériau hydrophobe, et
à travers la couche limitant l'infiltration de liquide (2), un liquide est amené à migrer de la couche perméable au liquide (1) vers la couche de rétention absorbante (3).

13. Pansement pour plaies selon la revendication 12, dans lequel la couche limitant l'infiltration de liquide (2) comprend une résine polyoléfinique et présente une perméabilité mesurée suivant la norme JIS L 1096 de 5 à 2000 cm³/cm²·S et un caractère hydrofuge mesuré suivant la norme JIS L 1092 de classe supérieure ou égale à 3.

14. Pansement pour plaies selon la revendication 12 ou 13, dans lequel la couche limitant l'infiltration de liquide (2) comprend un tissu non tissé comprenant une fibre de polypropylène.

15. Pansement pour plaies selon l'une quelconque des revendications 5 à 14, dans lequel la couche de rétention absorbante (3) comprend un tissu non tissé formé par voie pneumatique.

16. Pansement pour plaies selon l'une quelconque des revendications 5 à 15, dans lequel la couche de rétention absorbante (3) comprend une pâte en flocons.

17. Pansement pour plaies selon la revendication 16, dans lequel la couche de rétention absorbante (3) comprend en outre un polymère super absorbant, et le rapport massique du polymère super absorbant et de la pâte en flocons va de 10:90 à 25:75.

18. Pansement pour plaies selon la revendication 17, dans lequel le polymère super absorbant est un polymère polyacrylate de sodium.

19. Pansement pour plaies selon l'une quelconque des revendications 5 à 18, dans lequel la couche de retenue absorbante (3) est partiellement non jointe à une couche adjacente qui se trouve sur le côté plus proche du site de la plaie.

20. Pansement pour plaies selon la revendication 19, dans lequel la couche de rétention absorbante (3) n'est pas intégrée à la couche perméable au liquide (1) et est mobile le long de la seconde surface (12) de la couche perméable au liquide (1).

21. Pansement pour plaies comprenant une couche perméable au liquide (1) comprenant la feuille de surface (10) selon l'une quelconque des revendications 1 à 4.

22. Pansement pour plaies selon la revendication 21, dans lequel une couche limitant l'infiltration de liquide (2) est empilée sur la seconde surface (12) de la feuille de surface (10),
la couche limitant l'infiltration de liquide (2) comprend un film microporeux, un tissu tissé, un tissu maillé ou un tissu non tissé, chacun comprenant un matériau hydrophobe, et
la couche limitant l'infiltration de liquide (2) permet l'infiltration d'un liquide dans le sens de l'épaisseur.

23. Pansement pour plaies selon la revendication 22, dans lequel la couche limitant l'infiltration de liquide (2) comprend une résine polyoléfinique et une perméabilité mesurée suivant la norme JIS L 1096 de 5 à 2000 cm³/cm²·S et un caractère hydrofuge mesuré suivant la norme JIS L 1092 de classe supérieure ou égale à 3.

24. Pansement pour plaies selon la revendication 22 ou 23, dans lequel la couche limitant l'infiltration de liquide (2) comprend un tissu non tissé comprenant une fibre de polypropylène.

25. Pansement pour plaies selon l'une quelconque des revendications 5 à 24, comprenant une couche adhésive (21) sur la surface opposée à la surface qui doit faire face au site de la plaie (15).
